(19) **Europäisches Patentamt**
**European Patent Office**
**Office européen des brevets**

(11) **EP 2 700 424 B1**

(12) **EUROPEAN PATENT SPECIFICATION**

(45) Date of publication and mention
of the grant of the patent:
**10.01.2018  Bulletin 2018/02**

(51) Int Cl.:
*A61M 5/142* *(2006.01)*     *F04B 43/12* *(2006.01)*
*A61M 5/168* *(2006.01)*     *F04B 43/08* *(2006.01)*
*A61M 5/36* *(2006.01)*

(21) Application number: **12774397.9**

(22) Date of filing: **19.04.2012**

(86) International application number:
**PCT/JP2012/002720**

(87) International publication number:
**WO 2012/144219 (26.10.2012 Gazette 2012/43)**

(54) **INFUSION PUMP**

INFUSIONSPUMPE

POMPE DE PERFUSION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **19.04.2011   JP 2011093092
27.04.2011   JP 2011100073**

(43) Date of publication of application:
**26.02.2014   Bulletin 2014/09**

(73) Proprietor: **Terumo Kabushiki Kaisha
Tokyo 151-0072 (JP)**

(72) Inventors:
• **MOCHIZUKI, Tomonori
Ashigarakami-gun
Kanagawa 259-0151 (JP)**

• **UEMURA, Tomoko
Ashigarakami-gun
Kanagawa 259-0151 (JP)**
• **ISHIKAWA, Kou
Ashigarakami-gun
Kanagawa 259-0151 (JP)**

(74) Representative: **Gill, Stephen Charles
Mewburn Ellis LLP
City Tower
40 Basinghall Street
London EC2V 5DE (GB)**

(56) References cited:
**WO-A1-2009/133703     GB-A- 2 312 046
JP-A- 4 240 457     JP-A- 7 313 593
JP-A- 2003 286 959     JP-A- 2010 537 678
JP-B2- 3 320 179**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Technical Field

The present invention relates to an infusion pump for delivering a drug or the like to a patient.

### Background Art

An infusion pump is used in, for example, an intensive-care unit (ICU), to accurately carry out a drug delivery treatment for a patient for a relatively long time. The infusion pump has a main body and an opening and closing door. A predetermined drug containing bag (infusion bag) is placed above the infusion pump, and an infusion tube hanging from the infusion pump is sandwiched between the main body and the opening and closing door. The infusion tube is placed in the main body, and the opening and closing door is closed to hold the infusion tube.

In the main body of the infusion pump, an outer peripheral surface of the infusion tube set in place is sandwiched between an inner surface of the opening and closing door and a plurality of peristaltic fingers. The infusion pump is a peristaltic infusion pump that delivers a drug by individually driving a plurality of peristaltic fingers of a delivery driving unit so that the peristaltic fingers sequentially press an outer peripheral surface of an infusion tube along a length direction (see Patent Document 1).

In the infusion pump described in Patent Document 1, the infusion tube is passed through and held in the main body of the infusion pump from top to bottom of the main body in a vertical direction.

In contrast, an infusion pump has been proposed in which the infusion tube is passed through and held in the main body of the infusion pump in a horizontal direction. A reason for this attempt to adopt a structure in which the infusion tube is passed through and held in the main body of the infusion pump in the horizontal direction is as follows. That is, in contrast to the infusion pump in which the infusion tube is passed through the main body of the infusion pump from top to bottom of the main body in the vertical direction, the proposed structure is advantageous in that the infusion tube causes no obstruction even when a plurality of infusion pumps are held on top of one another so as to be stacked in an up-down direction.

### Citation List

### Patent Literature

Patent Document 1: Japanese Patent Application Laid-open No. 2010-200775
GB A 2312046 proposes an air in line ultrasonic detector for an IV tube.

### Summary of Invention

### Technical Problem

In the infusion pump in Patent Document 1, the infusion tube is set in the up-down direction, thus preventing an upstream side and a downstream side of the tube from being mixed up during setting.
However, in the infusion pump in which the infusion tube is passed through and held in the infusion pump in the horizontal direction, the infusion tube is set in the horizontal direction in the main body of the infusion pump. Thus, a health-care professional (user) may mix up the upstream side and the downstream side of the infusion tube and set the infusion tube in the opposite direction along the horizontal direction with respect to the main body of the infusion pump.
For example, if the infusion tube is predetermined to be arranged such that the upstream side of the infusion tube is placed on a right side portion of the main body of the infusion pump, whereas the downstream side of the infusion tube is placed on a left side portion of the main body of the infusion pump, as viewed from the health-care professional, when the upstream side of the infusion tube is placed on the right side portion of the main body of the infusion pump, whereas the downstream side of the infusion tube is placed on the left side portion of the main body of the infusion pump, the delivery driving unit is driven to allow a drug to be delivered from upstream side to downstream side along a predetermined delivery direction. As a result, the drug can be correctly delivered to the patient.
However, when the health-care professional incorrectly places the infusion tube in the opposite direction with respect to the main body, that is, when the health-care professional places the upstream side of the infusion tube on the left side portion of the main body of the infusion pump, while placing the downstream side of the infusion tube on the right side portion of the main body of the infusion pump, the drug is delivered in a direction opposite to the correct direction and thus fails to be correctly delivered to the patient.
Thus, an object of the present invention is to provide a infusion pump that allows the health-care professional to easily visually check whether the infusion tube is installed in an incorrect direction during installation of the infusion tube, to enable the drug to be correctly delivered from upstream side to downstream side of the infusion tube along the preset delivery direction.

### Solution to Problem

The present invention provides an infusion pump according to claim 1.
According to this configuration, when the clamp is opened to carry out priming, the control unit determines that the infusion tube is set in the direction opposite to the delivery direction based on the downstream occlusion signal (S3). Thus, the drug can be prevented from being subsequently delivered in the opposite direction.

Preferably, the infusion pump has warning means for issuing a warning in accordance with an instruction from the control unit when the control unit determines that the infusion tube is installed in the direction opposite to the delivery direction.

According to this configuration, if the health-care professional has incorrectly set the infusion tube in the direction opposite to the delivery direction with respect to the tube installation unit, the warning means issues a warning to allow the health-care professional to be informed of the incorrect setting of the infusion tube. The health-care professional can thus take appropriate measures.

The present invention can provide an infusion pump that prevents the health-care professional from installing the infusion tube in the incorrect direction during installation of the infusion tube, allowing the drug to be correctly delivered from upstream side to downstream side of the infusion tube along the preset delivery direction. The present invention can also provide a infusion pump that prevents the health-care professional from installing the infusion tube in the incorrect direction during installation of the infusion tube.

Brief Description of Drawings

Fig. 1 is a perspective view showing a first embodiment of an infusion pump according to the present invention.

Fig. 2 is a perspective view of the infusion pump shown in FIG. 1 as viewed in a W direction.

Fig. 3 is a perspective view showing a tube installation unit in which an infusion tube is installed by opening an opening and closing cover of the infusion pump shown in Fig. 1 and Fig. 2.

Fig. 4 is a front view of the infusion pump shown in Fig. 3 as viewed in a V direction.

Fig. 5 is a diagram showing an example of an electrical configuration of the infusion pump.

Fig. 6 is a diagram showing the infusion pump, an infusion bag, and an infusion tube disposed along a correct delivery direction with respect to the infusion pump.

Fig. 7(A) is a diagram showing that the infusion tube is set along a T direction, the correct direction with respect to the infusion pump, and Fig. 7(B) is a diagram showing that the infusion tube is set along an N direction, an incorrect direction with respect to the infusion pump.

Fig. 8(A) is a diagram showing a priming state before the infusion tube is connected to a patient, and Fig. 8(B) is a diagram showing that a drug is being delivered to the patient through the infusion tube, wherein, in both Fig. 8(A) and Fig. 8(B), the infusion tube is set in the N direction, the incorrect direction with respect to a tube installation unit.

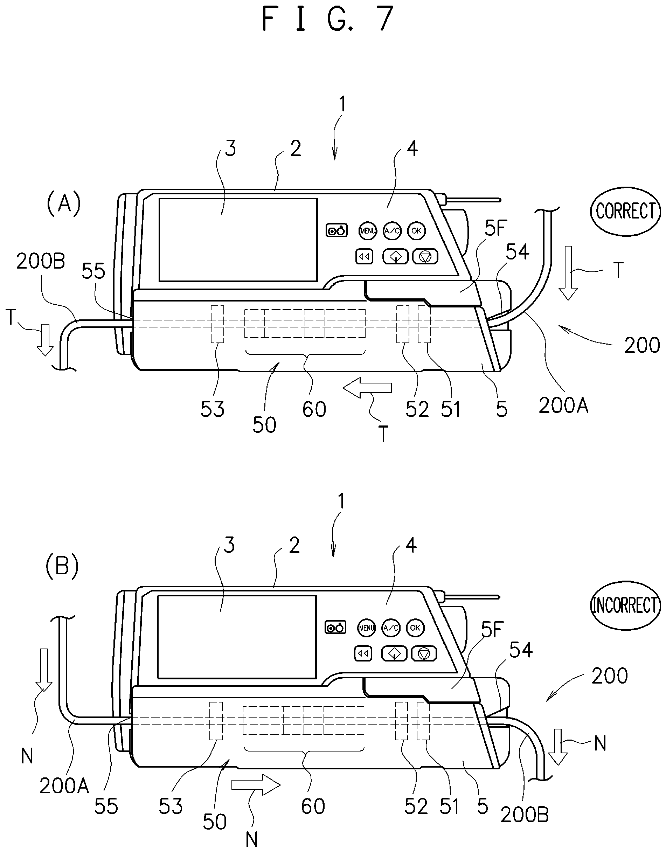

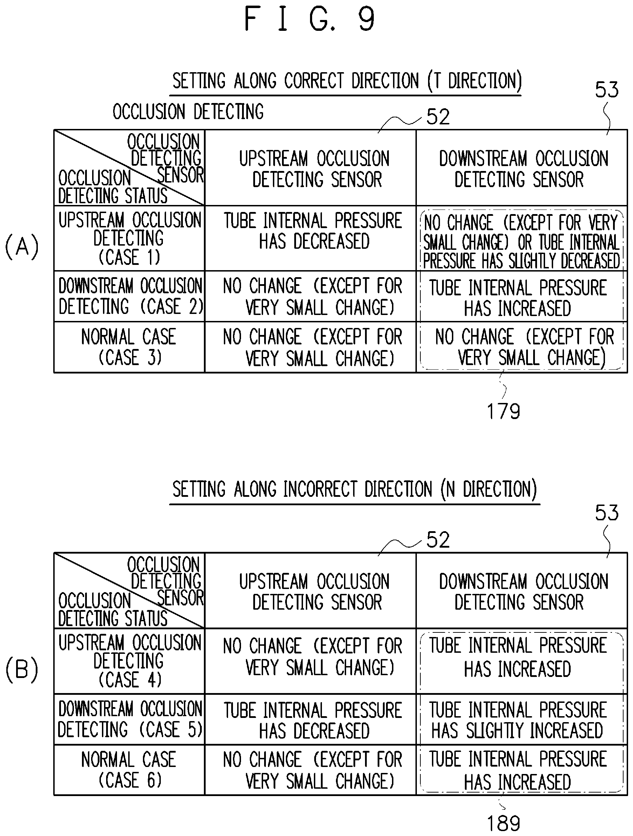

Fig. 9(A) shows a occlusion status of an infusion line and examples of contents of detection by an upstream occlusion sensor and a downstream occlusion sensor, the occlusion status and the detection being observed when the infusion tube is set along the T direction (forward direction), the correct direction, as shown in Fig. 7(A), and Fig. 9(B) shows the occlusion status of the infusion line and examples of contents of detection by the upstream occlusion sensor and the downstream occlusion sensor, the occlusion status and the detection being observed when the infusion tube is set along the N direction (opposite direction), the incorrect direction as shown in Fig. 7(B).

Fig. 10 is a diagram showing a flow illustrating an example of operation of the infusion pump.

Fig. 11 is a diagram showing examples of warning messages displayed on a display unit in Fig. 5.

Fig. 12 is a diagram showing an example in which a plurality of infusion pumps shown in Fig. 1 and Fig. 2 are mounted on an infusion stand.

Fig. 13 is a diagram of the appearance of an infusion pump according to a second embodiment.

Fig. 14 is a diagram showing that a door unit of the infusion pump is open.

Fig. 15 is a block diagram of an electric system in the infusion pump.

Fig. 16 is a diagram illustrating the principle of detection of opposite installation of an infusion tube according to the second embodiment.

Fig. 17 is a flowchart showing a process procedure of a control unit of the infusion pump according to the second embodiment.

Fig. 18 is a diagram illustrating the principle of detection of opposite installation of an infusion tube according to a third embodiment.

Fig. 19 is a flowchart showing a process procedure of a control section of an infusion pump according to the third embodiment.

Description of Embodiments

An first embodiment of the present invention will be described below with reference to the drawings.

Embodiments described below are preferred specific examples of the present invention. Thus, various technically preferred limitations are imposed on the embodiments, but the scope of the preset invention is not limited to these aspects unless the description below refers to a limitation on the present invention.

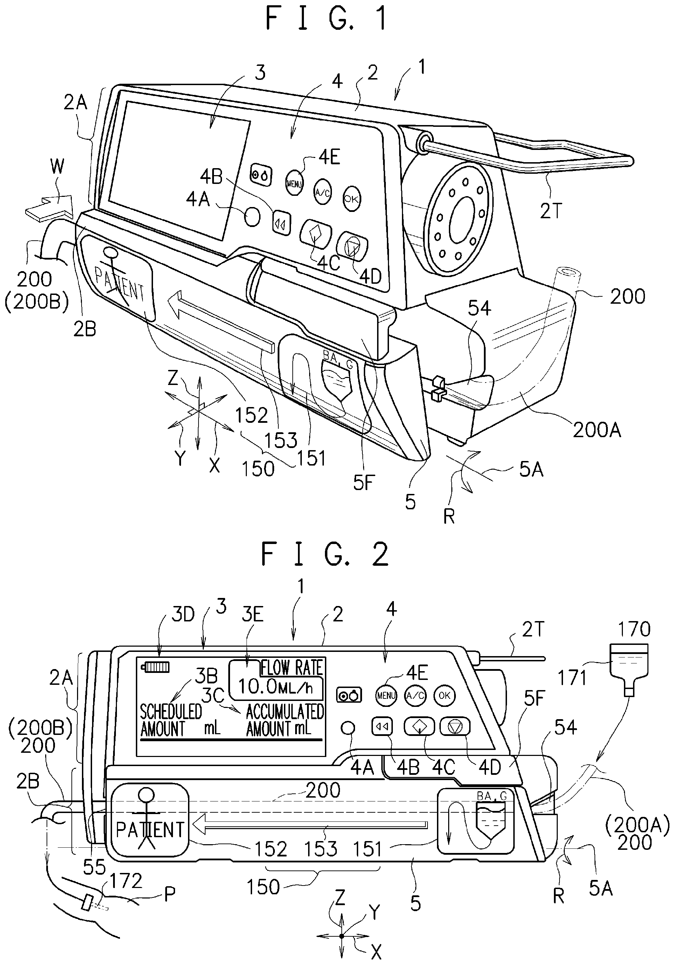

Fig. 1 is a perspective view showing a first embodiment of an infusion pump according to the present invention. Fig. 2 is a perspective view of the infusion pump shown in Fig. 1 as viewed in a W direction.

An infusion pump 1 shown in Fig. 1 and Fig. 2 is an infusion pump used in, for example, an intensive-care unit (ICU, CCU, or NICU) to infuse a drug, for example, an anticancer drug, an anesthetic, a chemical treatment drug, blood, or a nutrient at a rate of about 1 to 999 mL/h (the amount of drug infused per hour, that is, an infusion speed) to within ±2% for a relatively long time so that about 1 to 9,999 mL (the scheduled amount of drug infused) of drug is infused into the patient. In some cases, a drug for use is selected from a drug library, and the infusion pump 1 is used to deliver the selected drug. The drug library is drug information in a chemical library database (DB) which is indicative of a drug administration setting group including preregistered drug names. Using the drug library eliminates the need for a health-care professional to carry out complicated administration setting every time, and allows a drug to be selected and set.

As shown in Fig. 2, the infusion pump 1 can carry out accurate delivery from an infusion bag 170 filled with a drug 171 to a patient P via an infusion tube 200 and an indwelling needle 172. The drug is also referred to as an infusion fluid. The infusion tube is also referred to as an infusion line.

The infusion pump 1 has a main body cover 2 and a handle 2T. The main body cover 2 is integrally formed of a molding resin material with chemical resistance and has a splash proof structure that, even when the infusion pump 1 is splashed with a drug or the like, protects the infusion pump 1 from entry of the drug or the like into the infusion pump 1. The main body cover 2 thus has the splash proof structure because the drug 171 in the infusion bag 170 disposed above may drop out or an antiseptic solution used around the infusion pump may fly and attach to the infusion pump.

Elements disposed on the main body cover 2 of the infusion pump 1 will be described.

As shown in Fig. 1 and Fig. 2, a display unit 3 and an operation panel unit 4 are disposed in an upper portion 2A of the main body cover 2. The display unit 3 is an image display device, and for example, a color liquid crystal display device is used as the display unit 3. The display unit 3 is disposed at an upper left position in the upper portion 2A of the main body cover 2 and above an opening and closing cover 5.

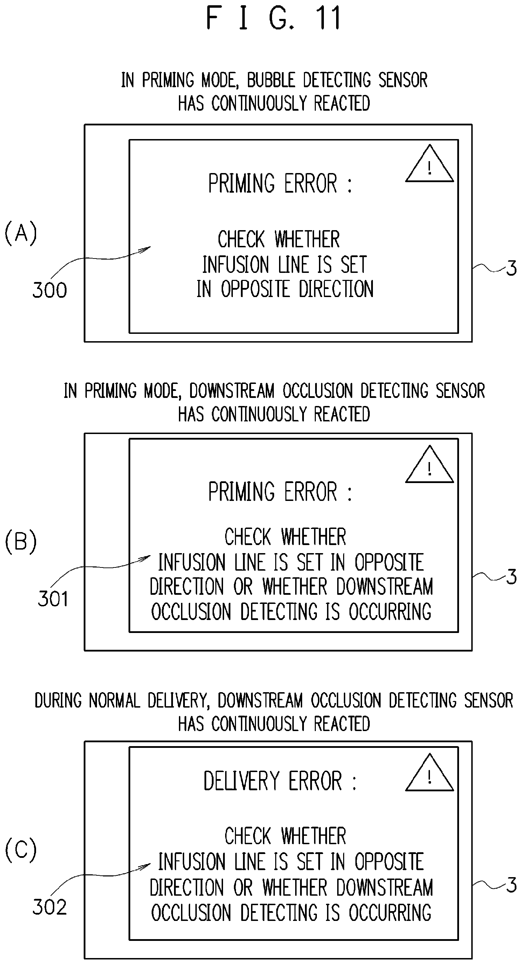

In Fig. 2, the display unit 3 displays, by way of example, a display section 3B for the scheduled amount of drug infused (mL), a display section 3C for the accumulated amount of drug administered (mL), a display section 3D for a history of charging, and a display section 3E for an infusion speed (mL/h). However, for simplification, the illustration of contents of these sections is omitted from the display unit 3 shown in Fig. 1. The display unit 3 may display such warning messages 300, 301, and 302 as shown in Fig. 11.

The operation panel unit 4 is disposed in the upper portion 2A of the main body cover 2 and in the right side of the display unit 3. The operation panel unit 4 has operation buttons, for example, in the illustrated example, a power supply switch button 4A, a fast feeding switch button 4B, a start switch button 4C, a stop switch button 4D, and a menu selection button 4E arranged thereon.

As shown in Fig. 1, the main body cover 2 includes the opening and closing cover 5 which is provided in a lower portion 2B of the main body cover 2 and which is openable and closable in an R direction around an axis of rotation 5A. The opening and closing cover 5 is a cover formed to be elongate along an X direction and configured to allow the infusion tube 200 formed of a flexible thermoplastic resin, for example, soft vinyl chloride to be installed horizontally along the X direction by placing the infusion tube 200 on an inner surface of the opening and closing cover 5 in a sandwiched manner.

As shown in Fig. 2, a surface of the opening and closing cover 5 is provided with an infusion tube set direction display unit 150 for definitely displaying a T direction, the correct delivery direction, set the infusion tube 200 is set. The infusion tube set direction display unit 150 has a drug containing bag display unit 151 that displays a drug containing bag side, a patient side display unit 152 that displays a patient side, and a delivery direction display unit 153 that definitely shows the delivery direction of the drug.

The drug containing bag display unit 151, the patient side display unit 152, and the delivery direction display unit 153 are, for example, printed on a seal material. The drug containing bag display unit 151, the patient side display unit 152, and the delivery direction display unit 153 are stuck to the surface of the opening and closing cover 5 at a position corresponding to a tube installation unit 50. Moreover, a small display unit with contents similar to those of the drug containing bag display unit 151 and the patient side display unit 152 may be stuck to an appropriate place in the tube installation unit 50. This allows immediate determination of whether or not an installation direction of the infusion tube 200 in the tube installation unit 50 is correct during installation of the infusion tube 200.

As shown in Fig. 2, the drug containing bag display unit 151 is disposed to allow visual checking of whether the drug containing bag 170 side of the infusion tube 200 is located on the right side portion of the opening and closing cover 5 as viewed from the health-care professional. The patient side display unit 152 is disposed to allow visual checking of whether the patient P side of the infusion tube 200 is located on the left side portion of the opening and closing cover 5 as viewed from the health-care professional. The delivery direction display unit 153 is disposed to definitely show the correct delivery direction (T direction) of the drug 171 through the infusion tube 200 set inside the opening and closing cover 5. The delivery direction display unit 153 is an arrow extending along the T direction and indicating a direction from the drug containing bag display unit 151 toward the patient side display unit 152. The drug containing bag display unit 151, which displays the drug containing bag side, and the patient side display unit 152, which displays the patient side, are both expressed at least in characters and preferably in characters and graphics. Thus, the health-care professional can be prevented from making a mistake in the setting direction of the infusion tube.

An X direction, a Y direction, and a Z direction in Fig. 1 and Fig. 2 are orthogonal to one another, and the Z direction is an up-down direction. The X direction is parallel to the T direction and is a lateral direction of the infusion pump 1. The Y direction is a front-back direction of the infusion pump 1.

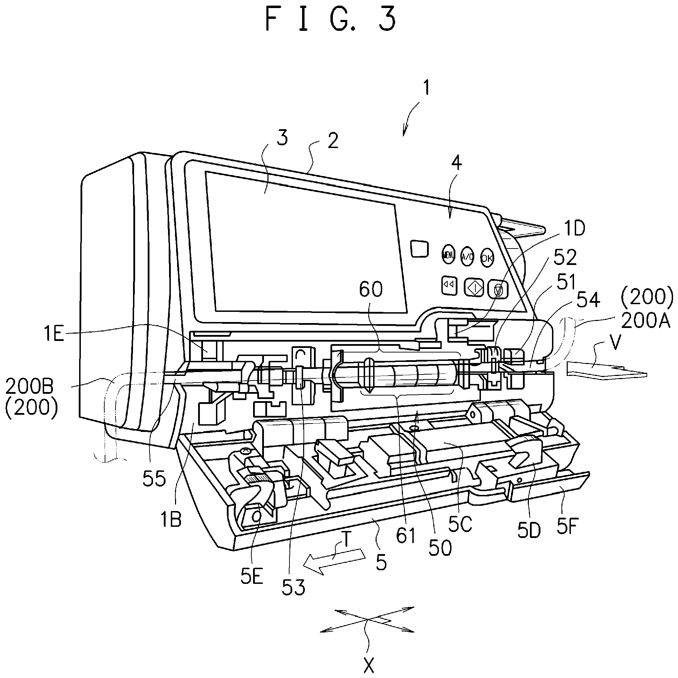

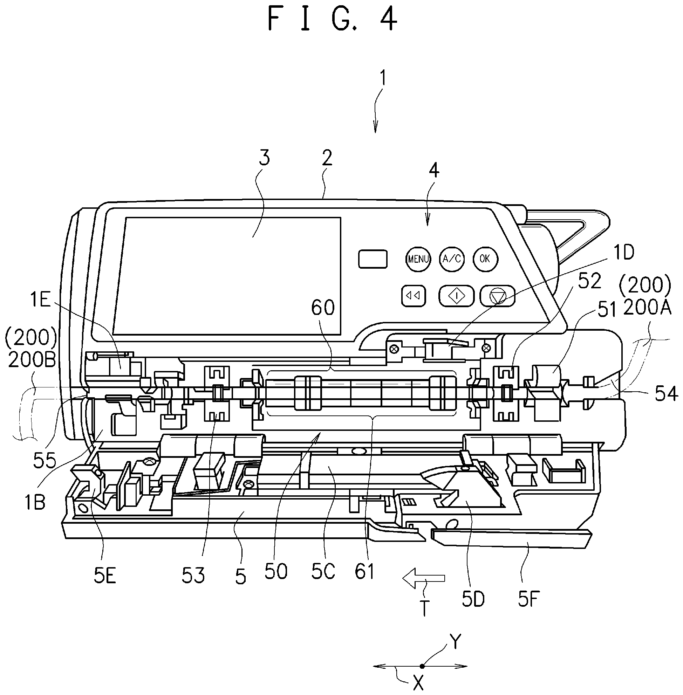

Fig. 3 is a perspective view showing the tube installation unit 50 in which the infusion tube 200 is installed by opening the opening and closing cover 5 of the infusion pump 1 shown in Fig. 1 and Fig. 2. Fig. 4 is a front view of the infusion pump 1 shown in Fig. 3 as viewed in a V direction.

As shown in Fig. 3 and Fig. 4, the tube installation unit 50 is provided on the main body unit 1B side of the infusion pump 1. The tube installation unit 50 is provided below the display unit 3 and the operation panel unit 4 along the X direction. The tube installation unit 50 can be covered with the opening and closing cover 5 when the opening and closing cover 5 is closed as shown in Fig. 2.

As shown in Fig. 3 and Fig. 4, the tube installation unit 50 has a bubble detecting sensor 51, an upstream occlusion sensor 52, a downstream occlusion sensor 53, a first infusion tube guide unit 54, a second infusion tube guide unit 55, and the opening and closing cover 5. An infusion tube presser member 5C and engagement members 5D and 5E are provided inside the opening and closing cover 5.

The infusion tube presser member 5C is arranged along the X direction as an elongate, rectangular, and planar projecting portion and positioned opposite a delivery driving unit 60. When a lever 5F interlocked the engagement members 5D and 5E is operated, the engagement members 5D and 5E are fitted into fitting portions 1D and 1E, respectively, of the main body unit 1B, allowing the opening and closing cover 5 to close the tube installation unit 50 of the main body unit 1B.

As shown in Fig. 4, a first infusion tube guide unit 54 is provided on the right side portion of the main body unit 1B as viewed from the health-care professional. A second infusion tube guide unit 55 is provided on the left side portion of the main body unit 1B as viewed from the health-care professional. The first infusion tube guide unit 54 can hold an upstream side 200A of the infusion tube 200 by fitting the upstream side 200A into the first infusion tube guide unit 54. The second infusion tube guide unit 55 can hold a downstream side 200B of the infusion tube 200 by fitting the downstream side 200B into the second infusion tube guide unit 55. The infusion tube 200 is held in the horizontal direction along the X direction. The infusion tube 200 thus installed (set) in the correct direction in the horizontal direction is sequentially disposed opposite the bubble detecting sensor 51, the upstream occlusion sensor 52, the delivery driving unit 60, and the downstream occlusion sensor 53 from the right side in Fig. 4.

In the main body unit 1B, the bubble detecting sensor 51, the upstream occlusion sensor 52, the delivery driving unit 60, and the downstream occlusion sensor 53 are arranged in this order between the first infusion tube guide unit 54 and the second infusion tube guide unit 55 along the T direction (parallel to the X direction). The delivery driving unit 60 is disposed between the upstream occlusion sensor 52 and the downstream occlusion sensor 53.

The bubble detecting sensor 51 shown in Fig. 4 is a sensor that detects bubbles (air) generated in the infusion tube 200. For example, the bubble detecting sensor 51 is an ultrasonic sensor that monitors bubbles contained in a drug flowing through the infusion tube 200 formed of soft vinyl chloride or the like, from the outside the infusion tube 200. An ultrasonic wave generated by a transmission unit of the ultrasonic sensor is applied to the drug flowing through the infusion tube 200. The transmittance of the ultrasonic wave through the drug is different from the transmittance of the ultrasonic wave through bubbles, and thus, a reception unit of the ultrasonic sensor detects a difference in transmittance to check whether or not bubbles are present.

The upstream occlusion sensor 52 shown in Fig. 4 is a sensor that detects whether the infusion tube 200 is blocked on the upstream side 200A thereof. The downstream occlusion sensor 53 is a sensor that detects whether the infusion tube 200 is blocked on the downstream side 200B thereof. As each of the upstream occlusion sensor 52 and the downstream occlusion sensor 53, for example, a photocoupler may be used which includes a combination of a light emission unit and a light reception unit. The light emission unit illuminates the infusion tube 200 with light, which is then received by the light reception unit. This allows the sensor to detect whether the infusion tube 200 is blocked.

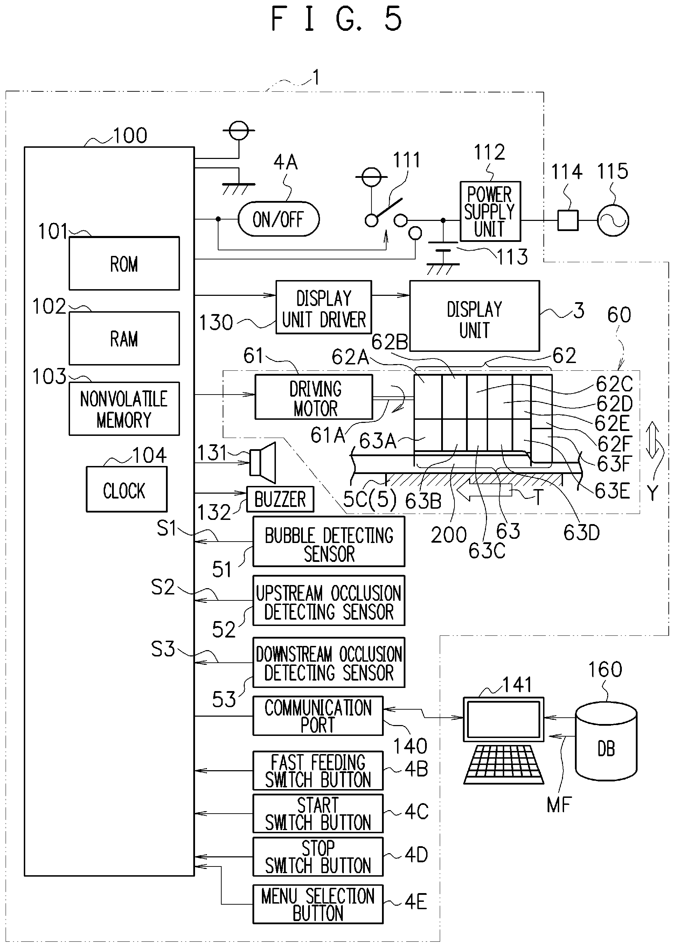

Fig. 5 shows an example of an electrical configuration of the infusion pump 1.

As shown in Fig. 5, the delivery driving unit 60 has a driving motor 61, a cam structure 62 with a plurality of cams rotationally driven by the driving motor 61, and a peristaltic finger structure 63 with a plurality of peristaltic fingers moved by the respective cams of the cam structure 62, as described below.

The cam structure 62 has a plurality of cams, for example, six cams 62A to 62F. The peristaltic finger structure 63 has six peristaltic fingers 63A to 63F corresponding to the six cams 62A to 62F. The six cams 62A to 62F are arrayed so as to differ from one another in phase. The cam structure 62 is connected to an output shaft 61A of the driving motor 61.

When the output shaft of the driving motor 61 rotates in accordance with an instruction from a control unit 100 shown in Fig. 5, the six peristaltic fingers 63A to 63F moves forward or backward in order in the Y direction by a predetermined stroke. Thus, the infusion tube 200 is pressed against the infusion tube presser member 5C of the opening and closing cover 5 along the T direction to allow the drug in the infusion tube 200 to be delivered in the T direction. That is, the plurality of peristaltic fingers 63A to 63F are individually driven to allow the plurality of peristaltic fingers 63A to 63F to sequentially project along the T direction to press an outer peripheral surface of the infusion tube 200, thus delivering the drug in the infusion tube 200. Peristaltic motion of the plurality of peristaltic fingers 63A to 63F is controlled to sequentially move the peristaltic fingers 63A to 63F forward or backward so that a blocked point on the infusion tube 200 is moved in the T direction in such a manner that wave motion progress in the T direction. Thus, the infusion tube 200 is squeezed to transfer the drug.

As shown in Fig. 5, the infusion pump 1 has the control unit (computer) 100 that controls general operation of the infusion pump 1. The control unit 100 is, for example, a one-chip microcomputer and has a ROM (Read Only Memory) 101, a RAM (Random Access Memory) 102, a nonvolatile memory 103, and a clock 104. The clock 104 allows the current time to be corrected by a predetermined operation and enables acquisition of the current time, measurement of elapsed time of a predetermined delivery operation, measurement of a reference time for delivery velocity control, and the like.

The control unit 100 shown in Fig. 5 connects to a power supply switch button 4A and a switch 111. The switch 111 switches between a power supply converter unit 112 and a rechargeable battery 113, for example, a lithium ion battery to supply power to the control unit 100 through the power supply converter unit 112 and the rechargeable battery 113. The power supply converter unit 112 is connected to a commercial AC power source 115 via an outlet 114.

The output shaft 61A of the driving motor 61 in the delivery driving unit 60 shown in Fig. 5 is rotated in accordance with an instruction from the control unit 100 to allow the plurality of peristaltic fingers 63A to 63F to make peristaltic motion. Thus, the infusion tube 200 is squeezed to transfer the drug in the T direction.

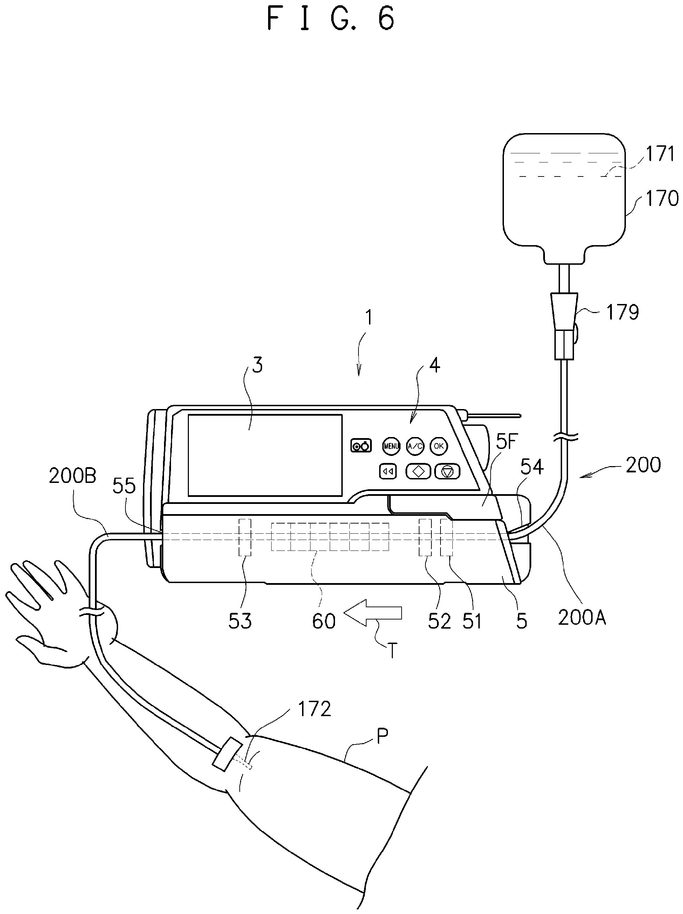

Fig. 6 shows the infusion pump 1, the drug containing bag 170, and the delivery tube 200 set along the T direction, the correct delivery direction with respect to the infusion pump 1.

As shown in Fig. 6, one end portion of the infusion tube 200 is connected to the infusion bag 170 via a clamp (clamp member) 179. The other end portion of the infusion tube 200 is connected to the indwelling needle 172 on the patient P side. The drug 171 in the infusion bag 170 is driven by the delivery driving unit 60 and delivered in the T direction, the correct direction, through the infusion tube 200 and the indwelling needle 172. The drug 171 is thus administered to the patient P.

Referring back to Fig. 5, a display unit driver 130 drives the display unit 3 in accordance with an instruction from the control unit 100 to display the contents of information illustrated in Fig. 2, warning messages 300 to 302 shown in Figure 11, and the like. A speaker 131 can provide an audio notification of the contents of various alarms in accordance with an instruction from the control unit 100. A buzzer 132 can provide an audio notification of various alarms in accordance with an instruction from the control unit 100. The speaker 131 is an example of warning means for issuing an audio warning to the health-care professional if the infusion tube 200 is set in the N direction (opposite direction). A buzzer 132 is an example of the warning means for issuing an audio warning to the health-care professional if the infusion tube 200 is set in the N direction (opposite direction), the incorrect direction.

In Fig. 5, the control unit 100 is provided with a bubble detection signal S1 from the bubble detecting sensor 51, an upstream occlusion signal S2 from the upstream occlusion sensor 52 which indicates that the upstream side of the infusion tube 200 is blocked, and a downstream occlusion signal S3 from the downstream occlusion sensor 53 which indicates that the downstream side of the infusion tube 200 is blocked. The upstream occlusion signal S2 from the upstream occlusion sensor 52 is a signal indicative of the magnitude of the internal pressure of the infusion tube 200 on the upstream side 200A. The downstream occlusion signal S3 from the downstream occlusion sensor 53 is a signal indicative of the magnitude of the internal pressure of the infusion tube 200 on the downstream side 200B.

The upstream occlusion sensor 52 and the downstream occlusion sensor 53 can detect a state in which the internal pressure of the infusion circuit exceeds a pressure set in the infusion pump 1 to preclude delivery of the drug, the alarms outputting the detected state to the control unit 100. Possible causes of the internal pressure of the infusion circuit exceeding the pressure set in the infusion pump 1 are occlusion of the indwelling needle or the infusion tube 200 for infusion, collapse or kink of the infusion tube 200, the use of a very viscous drug, and the like.

In Fig. 5, the control unit 100 can communicate with a computer 141, for example, a desktop computer, via a communication port 140 in a bidirectional manner. The computer 141 is connected to a drug database (DB) 160, and drug information MF stored in the drug database 160 can be provided to the control unit 100 and stored in the nonvolatile memory 103 of the control unit 100, via the computer 141. The control unit 100 can display the drug information MF and the like, for example, on the display unit 3 shown in Fig. 2 based on the stored drug information MF.

In Fig. 5, the fast feeding switch button 4B, the start switch button 4C, the stop switch button 4D, and the menu selection button 4E are electrically connected to the control unit 100.

Now, operation of the above-described infusion pump 1 during use will be described.

Fig. 7(A) shows that the infusion tube 200 is installed (set) along the T direction (forward direction), the correct direction with respect to the tube installation unit 50 of the infusion pump 1. Fig. 7(B) shows that the infusion tube 200 is set along the N direction (opposite direction), the incorrect direction with respect to the tube installation unit 50 of the infusion pump 1.

As shown in Fig. 3, the health-care professional, before opening the opening and closing cover 5 and setting the infusion tube 200 in the tube installation unit 50, views the infusion tube set direction display unit 150 disposed above the opening and closing cover 5 shown in Fig. 2 to visually check the set direction of the infusion tube 200. Thus, the infusion tube 200 can be correctly installed along the T direction, the delivery direction, allowing the drug 171 to be correctly delivered from the upstream side 200A to the downstream side 200B of the infusion tube 200 along the T direction, the preset delivery direction of the delivery driving unit 60.

As shown in Fig. 7(A), when the infusion tube 200 is set in the T direction, the correct direction, the upstream side 200A of the infusion tube 200 is disposed on the first infusion tube guide unit 54 side, the right side portion of the main body unit 1B as viewed from the health-care professional. The downstream side 200B of the infusion tube 200 is disposed on the second infusion tube guide unit 55 side, the left side portion of the main body unit 1B as viewed from the health-care professional. In the set state of the infusion tube 200 shown in Fig. 6 and Figs. 1 to 4, the infusion tube 200 is also correctly set to allow the drug to be delivered along the T direction.

In contrast, as shown in Fig. 7(B), when the infusion tube 200 is set in the N direction, the incorrect direction with respect to the infusion pump 1, the upstream side 200A of the infusion tube 200 is disposed at the second infusion tube guide unit 55, whereas the downstream side 200B of the infusion tube 200 is disposed at the first infusion tube guide unit 54. That is, the installation state of the infusion tube 200 is such that the health-care professional has mistaken the lateral arrangement positions of the upstream side 200A and downstream side 200B of the infusion tube 200 and installs the infusion tube 200 in the N direction, the incorrect direction with respect to the infusion pump 1. Thus, when the infusion tube 200 is incorrectly installed, the drug is delivered in the N direction, which is opposite to the T direction, the preset delivery direction of the delivery driving unit 60.

Thus, as shown in Fig. 7(A) and Fig. 2, to correctly install the infusion tube 200 along the T direction with respect to the infusion pump 1, the health-care professional first visually checks the drug containing bag display unit 151, which displays the drug containing bag side, the patient side display unit 152, which displays the patient side, and the delivery direction display section 153, which definitely shows the drug delivery direction, as shown in Fig. 2; the drug containing bag display unit 151, the patient side display unit 152, and the delivery direction display section 153 are all provided on the infusion tube set direction display unit 150 on the opening and closing cover 5. Thus, the health-care professional can position the upstream side 200A of the infusion tube 200 on the drug containing bag display unit 151 side, position the downstream side 200B of the infusion tube 200 on the patient side display unit 152, and visually check the set direction of the infusion tube 200 by means of the arrow of the delivery direction display section 153, which shows that the drug delivery direction is the T direction.

After viewing the drug containing bag display unit 151, which displays the drug containing bag side, the patient side display unit 152, which displays the patient side, and the delivery direction display section 153, which definitely shows the drug delivery direction, to check whether the infusion tube 200 is set in the T direction, the correct direction, as described above, the health-care professional operates the lever 5F to open the opening and closing cover 5 to expose the bubble detecting sensor 51, the upstream occlusion sensor 52, the downstream occlusion sensor 53, the first infusion tube guide unit 54, the second infusion tube guide unit 55, and the delivery driving unit 60, as shown in Fig. 3 and Fig. 4.

Then, the health-care professional installs the infusion tube 200 in the T direction along the first infusion tube guide unit 54, the bubble detecting sensor 51, the upstream occlusion sensor 52, the delivery driving unit 60, the downstream occlusion sensor 53, and the second infusion tube guide unit 55. Subsequently, as shown in Fig. 1 and Fig. 2, the opening and closing cover 5 is closed to cover the bubble detecting sensor 51, the upstream occlusion sensor 52, the downstream occlusion sensor 53, the first infusion tube guide unit 54, the second infusion tube guide unit 55, and the delivery driving unit 60. Thus, the infusion tube 200 can be installed in the T direction, the correct direction, and the drug can be delivered through the infusion tube 200 along the T direction by driving the delivery driving unit 60.

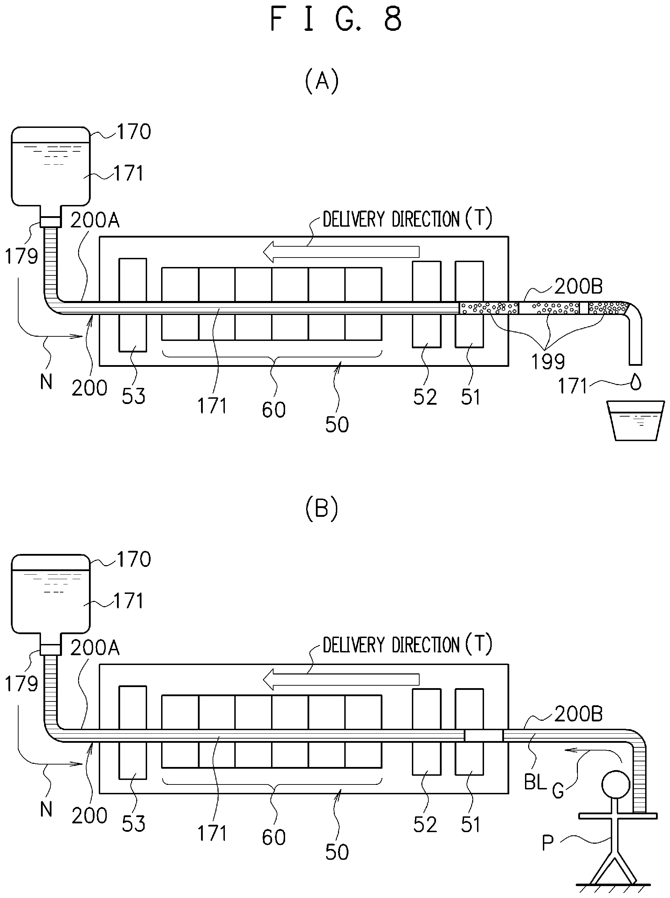

Fig. 8(A) shows a priming state before the infusion tube 200 is connected to the patient. Fig. 8(B) shows that the drug 171 in the infusion tube 200 is being delivered in an incorrect manner. In either Fig. 8 (A) or Fig. 8(B), the infusion tube 200 is installed in the N direction, the incorrect direction with respect to the tube installation unit 50. The priming refers to performing a preparatory operation in preparation for starting and to filling a drug into the infusion tube 200 to prepare for immediate connection to the indwelling needle placed in the vessel.

In the priming state before the infusion tube 200 is connected to the patient as shown in Fig. 8(A), the infusion tube 200 is installed in the N direction, the incorrect direction as also shown in Fig. 7(B). In this case, during the priming before the infusion tube 200 is connected to the patient, that is, while the fast feeding switch button 4B shown in Fig. 2 and Fig. 5 is being depressed and while the control unit 100 is driving the delivery driving unit 60 to fill the drug 171 into the infusion tube 200, the bubble detection signal S1 from the bubble detecting sensor 51, the upstream occlusion signal S2 from the upstream occlusion sensor 52, and the downstream occlusion signal S3 from the downstream occlusion sensor 53 have values different from values obtained when the infusion tube 200 is installed in the T direction, the correct direction as shown in Fig. 7(A). Utilizing this, the control unit 100 determines that the infusion tube 200 is installed in the N direction, the incorrect direction as shown in Fig. 7(B).

That is, when the priming is carried out if the infusion tube 200 is installed in the N direction, the incorrect direction as shown in Fig. 8(A), the bubbles 199 flow in toward the drug containing bag 170 through the end of the infusion tube 200. Consequently, the bubble detecting sensor 51 detects the bubbles 199 in the infusion tube 200. Thus, the bubble detecting sensor 51 transmits the bubble detection signal S1 to the control unit 100.

At this point, when the control unit 100 drives the speaker 131 and the buzzer 132 to issue, to the health-care professional, a warning indicating that the infusion tube 200 is installed in the N direction, the incorrect direction as shown in Fig. 7(B), the health-care professional can take appropriate measures to keep the patient safe.

Furthermore , in Fig. 8(B), when the priming before the infusion tube 200 is connected to the patient is carried out by cancelling the closed state of the clamp member 179, that is, when the control unit 100 fills the drug 171 into the infusion tube 200 through the drug containing bag 170 by cancelling the closed state of the clamp 179 of the infusion tube 200, instead of driving the delivery driving unit 60, the control unit 100, by focusing on the downstream occlusion signal S3 from the downstream occlusion sensor 53 during delivery, can determine based on the value of the downstream occlusion signal S3 that the infusion tube 200 is installed in the N direction, the incorrect direction as shown in Fig. 7(B).

If the infusion tube 200 is installed in the N direction, the incorrect direction as shown in Fig. 7(B), when the drug 171 is delivered from the drug containing bag 170 to the patient P through the infusion tube 200 as shown in Fig. 8(B), blood BL flows from the patient P toward the drug containing bag 170 through the infusion tube 200 in a G direction. Thus, the bubble detecting sensor 51 fails to detect bubbles and to transmit the bubble detection signal S1 to the control unit 100.

Thus, the following tendency is exploited: if the infusion tube 200 is installed in the N direction, the incorrect direction as shown in Fig. 8(B), while the drug 171 is being delivered to the patient through the infusion tube 200, the upstream occlusion signal S2 from the upstream occlusion sensor 52 and the downstream occlusion signal S3 from the downstream occlusion sensor 53 have values different from the values obtained when the infusion tube 200 is installed in the T direction, the correct direction. And, one of the value of the upstream occlusion signal S2 from the upstream occlusion sensor 52 and the value of the downstream occlusion signal S3 from the downstream occlusion sensor 53, that is, the value of the downstream occlusion signal S3, is focused on and utilized. By focusing on a tendency toward an increase in the internal pressure of the infusion tube 200 on the downstream side to check the downstream occlusion signal S3, the control unit 100 can determine that the infusion tube 200 is installed in the N direction, the incorrect direction.

Fig. 9(A) shows the occlusion status of the infusion tube 200 and examples of the contents of detection by the upstream occlusion sensor 52 and the downstream occlusion sensor 53, the status and the contents being obtained when the infusion tube 200 is installed in the T direction, as illustrated in Fig. 7(A). The contents of the detection are the same both during priming and during delivery.

In Fig. 9(A), when the infusion tube 200 is blocked on the upstream side 200A as in case 1, the upstream occlusion sensor 52 detects that the internal pressure of the upstream side 200A of the infusion tube 200 has decreased. The downstream occlusion sensor 53 detects that the internal pressure of the downstream side 200B of the infusion tube 200 has shown no substantial change (except for a very small change) or has decreased slightly. The upstream occlusion sensor 52 and the downstream occlusion sensor 53 notify the control unit 100 in Fig. 5 of the contents of the detection.

In Fig. 9(A), when the infusion tube 200 is blocked on the downstream side 200B as in case 2, the upstream occlusion sensor 52 detects that the internal pressure of the upstream side 200A of the infusion tube 200 has shown no substantial change (except for a very small change), and the downstream occlusion sensor 53 detects that the internal pressure of the downstream side 200B of the infusion tube 200 has increased. The upstream occlusion sensor 52 and the downstream occlusion sensor 53 notify the control unit 100 in Fig. 5 of the contents of the detection.

In Fig. 9(A), when neither the upstream side 200A nor the downstream side 200B of the infusion tube 200 is blocked and normal infusion is being carried out as in case 3, the upstream occlusion sensor 52 detects that the internal pressure of the upstream side 200A of the infusion tube 200 has shown no substantial change (except for a very small change), and the downstream occlusion sensor 53 detects that the internal pressure of the downstream side 200B of the infusion tube 200 has shown no substantial change (except for a very small change). The upstream occlusion sensor 52 and the downstream occlusion sensor 53 notify the control unit 100 in Fig. 5 of the contents of the detection.

In contrast, Fig. 9(B) shows the occlusion status of the infusion tube 200 and examples of the contents of detection by the upstream occlusion sensor 52 and the downstream occlusion sensor 53, the status and the contents being obtained when the infusion tube 200 is installed in the N direction, the incorrect direction, as illustrated in Fig. 7(B).

In Fig. 9(B), when the infusion tube 200 is blocked on the upstream side 200A as in case 4, the upstream occlusion sensor 52 detects that the internal pressure of the downstream side 200B of the infusion tube 200 has shown no substantial change (except for a very small change), and the downstream occlusion sensor 53 detects that the internal pressure of the upstream side 200A of the infusion tube 200 has increased. The upstream occlusion sensor 52 and the downstream occlusion sensor 53 notify the control unit 100 in Fig. 5 of the contents of the detection.

In Fig. 9(B), when the infusion tube 200 is blocked on the downstream side 200B as in case 5, the upstream occlusion sensor 52 detects that the internal pressure of the downstream side 200B of the infusion tube 200 has decreased. The downstream occlusion sensor 53 detects that the internal pressure of the upstream side 200A of the infusion tube 200 has increased slightly. The upstream occlusion sensor 52 and the downstream occlusion sensor 53 notify the control unit 100 in Fig. 5 of the contents of the detection.

In Fig. 9(B), when neither the upstream side 200A nor the downstream side 200B of the infusion tube 200 is blocked and normal operation is being performed as in case 6, the upstream occlusion sensor 52 detects that the internal pressure of the downstream side 200B of the infusion tube 200 has shown no substantial change (except for a very small change), and the downstream occlusion sensor 53 detects that the internal pressure of the upstream side 200A of the infusion tube 200 has increased. The upstream occlusion sensor 52 and the downstream occlusion sensor 53 notify the control unit 100 in Fig. 5 of the contents of the detection.

Thus, unlike in the case where the infusion tube 200 is installed in the T direction, the correct direction as shown in Fig. 9(A) and Fig. 7(A), in the case where the infusion tube 200 is installed in the N direction, the incorrect direction as shown in Fig. 9(B) and Fig. 7(B), the control unit 100 can recognize that the downstream occlusion sensor 53 detects a tendency toward an increase in the internal pressure of the infusion tube 200 in all of case 4 of the upstream occlusion, case 5 of the downstream occlusion, and case 6 of the normal infusion, as shown in a dashed frame 189 in Fig. 9(B).

In contrast, in a dashed frame 179 shown in Fig. 9(A), the internal pressure increases only in case 2 of the downstream occlusion but does not increase in case 1 of the upstream occlusion or case 3 of the normal infusion.

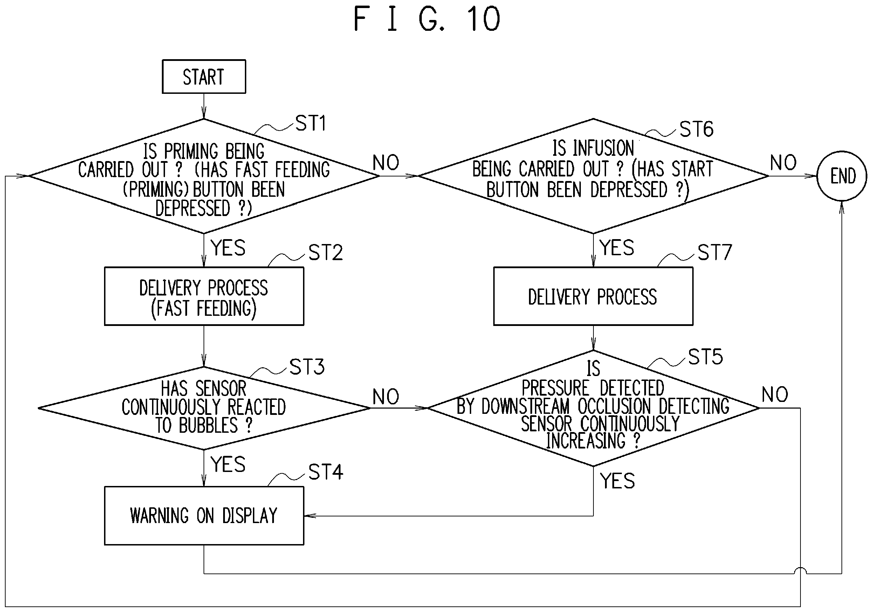

Fig. 10 is a flow diagram showing an example of operation of the infusion pump 1.

In step ST1 in Fig. 10, the control unit 100 shown in Fig. 5 determines whether the infusion pump 1 is in a priming mode or in a normal infusion mode. When the health-care professional keeps the fast feeding switch button, shown in Figure 5, depressed, the control unit 100 can determine that the infusion pump 1 is in the priming mode. When the result of the determination indicates that the infusion pump 1 is in the priming mode, the process shifts to step ST2 in Fig. 10, where the control unit 100 allows the delivery driving unit 60 to perform a fast feeding delivery operation. Thus, the drug is fed into the infusion tube 200 at high speed. At this point, it is unknown whether the infusion tube 200 is correctly set as shown in Fig. 7(A) or incorrectly set as shown in Fig. 7(B).

In step ST3 in Fig. 10, the control unit 100 checks whether the bubble detecting sensor 51 is continuously detecting the bubbles 199 in the infusion tube 200 as shown in Fig. 8(A). In step ST3, if the bubble detecting sensor 51 is continuously detecting the bubbles 199 in the infusion tube 200, the infusion tube 200 may be installed in the N direction, the incorrect direction, and the drug 171 may be flowing backward through the infusion tube 200.

Thus, in step ST4 in Fig. 10, by driving at least one of the speaker 131 and the buzzer 132, the control unit 100 in Fig. 5 can issue a warning to the health-care professional to notify, by at least one of voice announcement and a warning sound, the health-care professional that the infusion tube 200 is installed in the N direction, the incorrect direction as shown in Fig. 7(B). The warning allows the health-care professional to take appropriate measures. During the warning, the warning message 300 shown in Fig. 11(A) is displayed on the display unit 3 shown in Fig. 2.

On the other hand, in step ST3 in Fig. 10, if the bubble detecting sensor 51 fails to continuously detect the bubbles 199 in the infusion tube 200 as shown in Figure 8(A), the process shifts to step ST5 in Fig. 10. In step ST5, the control unit 100 in Fig. 5 checks whether the internal pressure of the infusion tube 200 detected by the downstream occlusion sensor 53 is continuously increasing. In step ST5 in Fig. 10, if the control unit 100 determines whether the internal pressure of the infusion tube 200 detected by the downstream occlusion sensor 53 is continuously increasing, the following two cases are possible.

In a first case, the infusion tube 200 is installed in the N direction, the incorrect direction, as shown in Fig. 7(B) (see cases 4, 5, and 6 in Fig. 9(B)).

In a second case, the infusion tube 200 is installed in the T direction, the correct direction, as shown in Fig. 7(A) but downstream occlusion is occurring in the infusion tube 200 (see case 2 in Fig. 9(A)).

In this case, the process shifts to step ST4 in Fig. 10, where a warning is issued which indicates that the infusion tube 200 is installed along the N direction, the incorrect direction, as shown in Fig. 7(B) or that the infusion tube 200 is installed in the T direction, the correct direction as shown in Fig. 7(A), but a downstream occlusion phenomenon may be occurring in the infusion tube 200. Furthermore, a warning message 301 shown in Fig. 11(B) is displayed on the display unit 3 in Fig. 5. The control unit 100 drives at least one of the speaker 131 and the buzzer 132. The health-care professional can be warned by at least one of the voice announcement and the warning sound as well as the warning message 301 on the display unit 3. The warning allows the health-care professional to take appropriate measures.

Referring back to step ST1 in Fig. 10, when the health-care professional has not depressed the fast feeding switch button 4B shown in Fig. 5 and the health-care professional depresses the start switch button 4C shown in Fig. 5, the control unit 100 determines that the infusion pump 1 is in the normal infusion mode and shifts from step ST6 to step ST7.

In step ST7, the control unit 100 in Fig. 5 allows the delivery driving unit 60 to perform a normal delivery operation to deliver the drug. In step ST5, the control unit 100 checks whether the internal pressure of the infusion tube 200 detected by the downstream occlusion sensor 53 is continuously increasing. In step ST5 in Fig. 10, if the control unit 100 determines that the internal pressure of the infusion tube 200 detected by the downstream occlusion sensor 53 is continuously increasing, the following two cases are possible.

In a first case, the infusion tube 200 is installed in the N direction, the incorrect direction, as shown in Fig. 7(B).

In a second case, the infusion tube 200 is installed in the T direction, the correct direction, as shown in Fig. 7(A) but downstream occlusion is occurring in the infusion tube 200.

In this case, the process shifts to step ST4 in Fig. 10, where a warning is issued which indicates that the infusion tube 200 is installed along the N direction, the incorrect direction, as shown in Fig. 7(B) or that the infusion tube 200 is installed in the T direction, the correct direction as shown in Fig. 7(A), but a downstream occlusion phenomenon may be occurring in the infusion tube 200. The control unit 100 drives at least one of the speaker 131 and the buzzer 132. Furthermore, a warning message 302 shown in Fig. 11(C) is displayed on the display unit 3 in Fig. 5. The health-care professional can be warned by at least one of the voice announcement and the warning sound as well as the warning message 302 on the display unit 3. The warning allows the health-care professional to take appropriate measures.

In step ST6 in Fig. 10, if the health-care professional does not depress the start switch button 4C shown in Fig. 5, no subsequent operation is performed. After the warning is issued in step ST4, no subsequent operation is performed.

As described above, according to the infusion pump 1 of the first embodiment of the present invention, the health-care professional, by viewing the infusion tube set direction display unit in setting the infusion tube, can visually check the upstream side and the downstream side of the infusion tube. Thus, the health-care professional is prevented from installing the infusion tube in the incorrect direction during installation of the infusion tube, allowing the drug to be correctly delivered from upstream side to downstream side of the infusion tube along the preset delivery direction.

Fig. 11 shows examples of the warning messages 300, 301, and 302 displayed on the display unit 3 in Fig. 5. The display unit 3 in Fig. 5 is an example of warning means for displaying the contents of a warning to visually warn the health-care professional when the infusion tube 200 is set in the N direction (opposite direction), the incorrect direction.

The warning message 300 shown in Fig. 11(A) is an example corresponding to an error in which, in the priming mode, the bubble detecting sensor 51 continuously detects and reacts to bubbles. The warning message 300 is displayed on the display unit 3 when step ST3 in Fig. 10 shifts to step ST4. The displayed warning message 300 is "priming error: check whether the infusion tube (infusion line) 200 is installed in the opposite direction".

The warning message 301 shown in Fig. 11(B) is an example corresponding to an error in which, in the priming mode, the downstream occlusion sensor 53 continuously reacts to downstream occlusion of the infusion tube 200. The warning message 301 is displayed on the display unit 3 when step ST3 in Fig. 10 shifts via step ST5 to step ST4. The displayed warning message 301 is "priming error: check whether the infusion tube (infusion line) 200 is installed in the opposite direction and whether downstream occlusion is occurring".

The warning message 302 shown in Fig. 11(C) is an example corresponding to an error in which, during normal delivery, the downstream occlusion sensor 53 continuously reacts to downstream occlusion of the infusion tube 200. The warning message 302 is displayed on the display unit 3 when step ST7 in Fig. 10 shifts via step ST5 to step ST4. The displayed warning message 301 is "delivery error: check whether the infusion tube (infusion line) 200 is installed in the opposite direction and whether downstream occlusion is occurring".

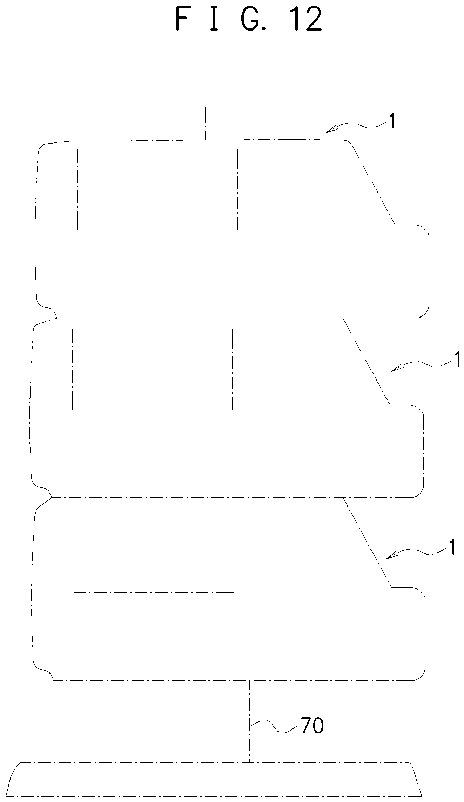

Fig. 12 shows an example in which a plurality of the infusion pumps 1 shown in Fig. 1 and Fig. 2 is mounted on a stand 70 so that the plurality of infusion pumps 1 can be simultaneously used as necessary. The infusion pump 1 allows the infusion tube 200 to be horizontally installed along the X direction. Thus, the plurality of infusion pumps 1 can be more easily laid on top of one another in the up-down direction and is thus more convenient than infusion pumps of a conventional structure in which the infusion tube is installed from an upper side to a lower side in the vertical direction.

The present invention is not limited to the above-described embodiment, can be variously modified and changed, and can be varied within the scope of the invention set forth in the claims.

In the infusion pump 1 according to the first embodiment of the present invention, a surface of the infusion tube set direction display unit 150 has the drug containing bag display unit 151, disposed on one end portion side of the opening and closing cover 5, the patient side display unit 152, disposed on the other end portion side of the opening and closing cover 5, and the delivery direction display section 153, which definitely shows the T direction, as shown in Fig. 2.

However, the present invention is not limited to this configuration. The infusion tube set direction display unit 150 may be disposed on the main body cover 2 side except for the opening and closing cover 5 rather than on the surface of the opening and closing cover 5. The arrangement position of the infusion tube set direction display unit 150 may be optionally selected.

Furthermore, the infusion tube set direction display unit 150 may be an "arrow" on, for example, any of the inner surfaces exposed when the opening and closing cover is opened, for example, at a position above the bubble detecting sensor 51 in Fig. 4.

In the example illustrated in Fig. 1 and Fig. 2, the infusion tube 200 is installed in the tube installation unit 50 along the T direction, which is the horizontal direction. However, the present invention is not limited to this configuration. For example, the tube installation unit 50 may adopt a structure in which the infusion tube 200 is installed in the lateral direction so as to incline downward from the upstream side 200A to the downstream side 200B by a predetermined angle.

Now, a second embodiment will be described.

[Description of the apparatus configuration]

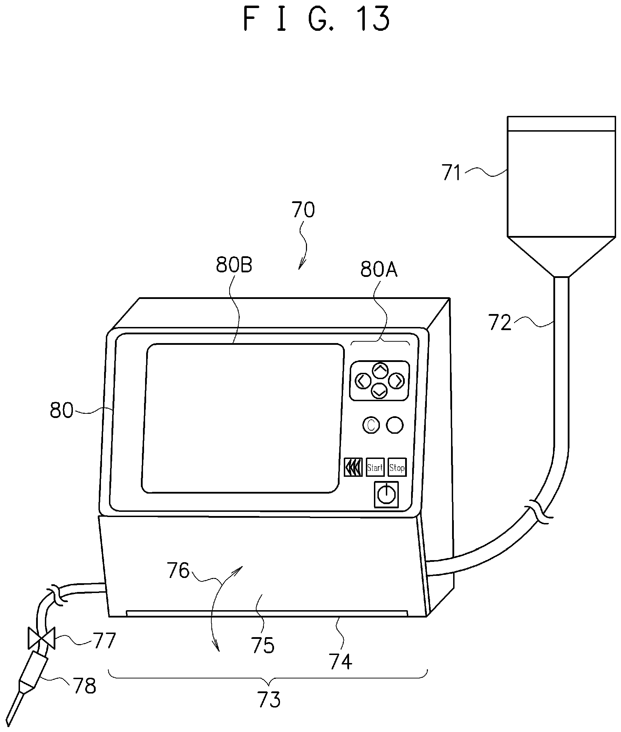

Fig. 13 is a diagram showing an example of an external configuration of an infusion pump 70 according to a second embodiment in which an infusion tube 72 from an infusion bag 71 is fixed in the correct direction. That is, in the description below, a right side of a front surface of the infusion pump 70 according to the second embodiment corresponds to an upstream side (infusion bag side). A left side of the front surface of the infusion pump 70 according to the second embodiment corresponds to a downstream side (patient side). The delivery direction is from right to left.

As shown in Fig. 13, an infusion bag 71 with a predetermined fluid medicine accommodated therein is connected to a delivery-direction upstream side of the infusion tube 72. Furthermore, an infusion clamp (clamp member) 77 and a vein puncture needle 78 are connected to a delivery-direction downstream side of the infusion tube 72. The vein puncture needle 78 is stuck into the patient's vein and placed therein to allow the fluid medicine in the infusion bag 71 to be infused into the patient. As shown in Fig. 13, in the infusion pump according to the present embodiment, the infusion tube 72 is accommodated in the horizontal direction and thus prevented from being folded even when other medical apparatuses are installed above and below the infusion pump in proximity thereto.

A front surface of the infusion pump 70 includes a door unit 75 that covers a delivery unit 73 in which the infusion tube 72 can be installed in the horizontal direction, and a user interface unit 80 disposed above the delivery unit 73 and the door unit 75.

The door unit 75 is connected to the delivery unit 73 at a hinge 74 and configured to be openable and closable in the direction of arrow 76 (opened and closed in the up-down direction).

The user interface unit 80 includes an operation unit 80A and a display unit 80B. The operation unit 80A includes operation switches via used to input various set values used by the delivery unit 73 for delivery and to give instructions to start and end delivery and to carry out priming or the like, and a power supply switch used to give an instruction to power the infusion pump 70 on and off.

Furthermore, the display unit 80B displays the various set values input via the operation unit 80A and the delivery status of the delivery unit 73.

In the infusion pump 70, the user interface unit 80 is disposed independently of the door unit 75. Thus, regardless of whether the door unit 75 is open or closed, the user can view the display unit 80B and perform various operations via the operation unit 80A.

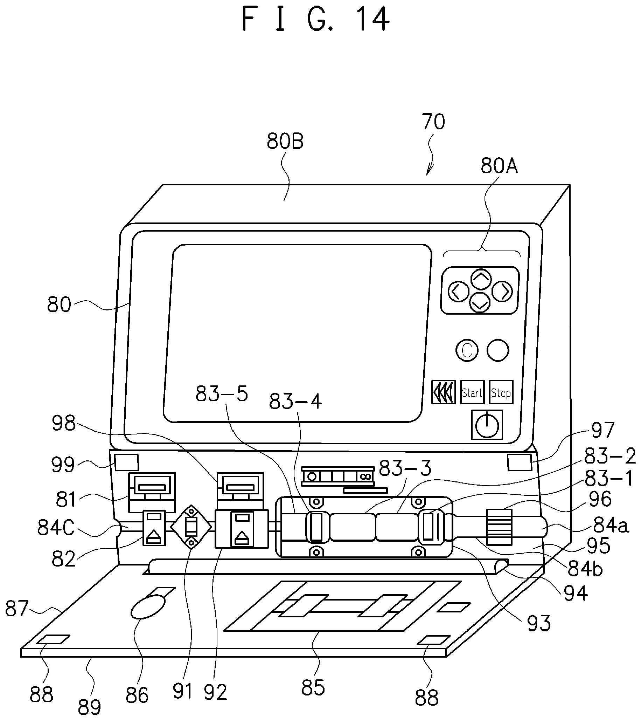

Now, a configuration of the delivery unit 73 of the infusion pump 70 will be described. Fig. 14 is a diagram showing an external configuration of the infusion pump 70, with the door unit 75 open and with the infusion tube removed therefrom.

In the door unit 75 in Fig. 14, the reference numeral 87 denotes a door base rotationally movably connected to a main body base 95 of the delivery unit 73 via the hinge unit 74. Hence, the door unit 75 is freely openable and closable with respect to the delivery unit 73.

The reference numeral 85 is a buffer plate mechanism that supports a rear surface of the infusion tube when the infusion tube is pressed by peristaltic fingers described below while the door unit 75 is closed. 86 is a occlusion presser plate that sandwiches the infusion tube between the occlusion presser plate and a occlusion sensor described below while the door unit 75 is closed.

In the delivery unit 73 in Fig. 14, the reference numeral 84 denotes a housing path in which the infusion tube is accommodated and which has a channel structure in which the infusion tube is housed and kept horizontal from right end to left end thereof as shown in Fig. 14.

The reference numeral 96 is a sensor channel unit including a bubble detecting sensor (not shown in the drawings) disposed on a sidewall unit forming the sensor channel unit 96, the bubble detecting sensor detecting bubbles in the infusion tube using ultrasonic wave or the like.

The reference numeral 93 is a pump mechanism including a plurality of peristaltic fingers 83-1 to 83-5 arrayed in the delivery direction and sequentially pressing the infusion tube.

The reference numeral 91 is a occlusion sensor including a permanent magnet and a pickup for detecting the amount of movement of the permanent magnet in an analog manner. The occlusion sensor 91 detects a blocked state by detecting the amount of movement of the permanent magnet, which moves in response to a change in internal pressure associated with the blocked state of the infusion tube.

The reference numeral 82 is a tube clamp holding unit that holds a clamp attached to the infusion tube and which applies a pressing force for temporarily closing the infusion tube under pressure, to the clamp when the door unit 75 is opened.

The tube clamp holding unit 82 is configured to be able to detect that the clamp attached to the infusion tube has been held. This allows determination of whether or not the infusion tube is installed in the infusion pump 70.

The reference numeral 81 is a lock/unlock lever that is operated to add the pressing force to the clamp of the infusion tube by the tube clamp holding unit 82 and to release the additional pressing force (that is, cancel the pressurized closure of the infusion tube by the clamp).

The tube clamp holding unit 82 may be configured to be able to detect that the clamp attached to the infusion tube has been held. This may allow determination of whether or not the infusion tube is installed in the infusion pump 70.

The reference numeral 92 is an infusion tube opposite-installation detection unit (described below in detail). 98 is a lock/unlock lever that holds and releases the infusion tube in and from the infusion tube opposite-installation detection unit in order to maintain a state in which the infusion tube is held in the infusion tube opposite-installation detection unit 92 and to remove the infusion tube.

Reference numerals 88 and 97 are an attraction unit such as a magnet and an attracted unit, respectively, which attract each other to keep the door unit 75 closed. The attraction unit 88 and the attracted unit 97 are configured to allow detection of whether or not the attraction unit and the attracted unit are attracting each other. This allows determination of whether the door unit 75 of the infusion pump 70 is open or closed. 88 may be a hook and 97 may be shaped to engage with the hook.

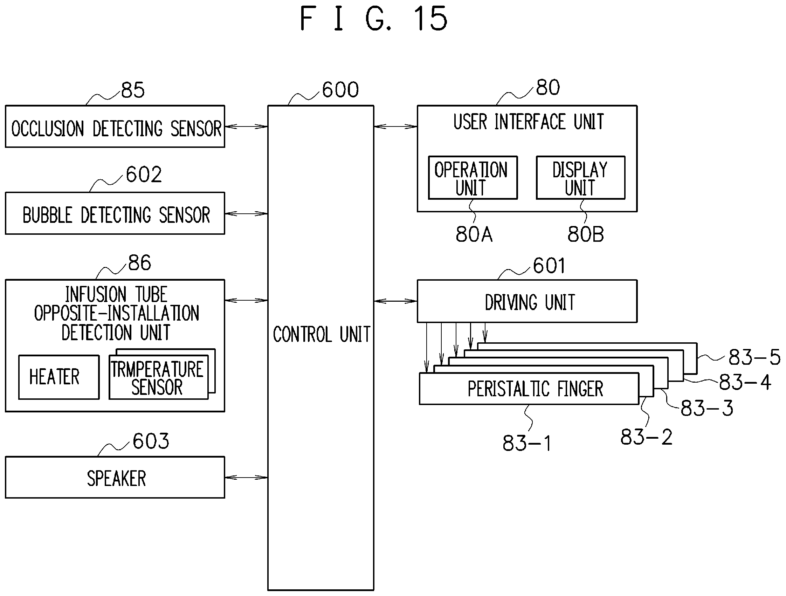

Fig. 15 is a block diagram of an electric system in the infusion pump according to the second embodiment.

In Fig. 15, reference numeral 600 is a control unit which controls the whole apparatus and which includes a microprocessor, a ROM storing programs that are process procedures for the microprocessor, and a RAM used as work areas. Reference numeral 601 is a driving unit that drives peristaltic fingers 83-1 to 83-5. When the peristaltic fingers 83-1 to 83-5 are cyclically driven to press the infusion tube in turn, the fluid medicine in the infusion tube can be delivered from right side to left side of Fig. 14. Reference numeral 602 is a bubble detecting sensor disposed near the sensor unit 96 in Figure 14 to detect whether the infusion tube accommodated in the sensor channel unit 96 is filled with the fluid medicine or mixed with bubbles as described above.

A general operation procedure for the above-described configuration will be described below.

First, the door unit 75 of the infusion pump 70 is opened, an operation of placing the tube from the infusion bag in housing paths 84a, 84b, and 84c is performed, and the door unit 75 is closed. Subsequently, the power supply switch on the user interface 80 is operated to power the infusion pump 70 on. Then, the clamp 77 is opened, and the priming switch provided on the operation unit 80A is operated. As a result, the control unit 600 controllably drives the driving unit 601 and drives the peristaltic fingers 83-1 to 83-5 to fill the fluid medicine from the infusion bag 71 into the tube 72.

Subsequently, while viewing the operation unit 80B, the operator operates the operation unit 80A to set the flow rate of infusion per unit time (mL/h) and the amount of fluid medicine infused (mL) (determine a driving period for the peristaltic fingers 83-1 to 83-5) . Then, the operator sticks the vein puncture needle 78 into the patient's vein and operates the infusion start switch on the operation unit 80A.

During this infusion process, when the bubble detecting sensor 602 detects bubbles or the occlusion sensor 91 detects occlusion, the warning message is blinked on the display unit 80A and the speaker 603 generates an alarm sound to notify the operator of the bubbles or the occlusion.

Moreover, according to the second embodiment, when an instruction to start infusion is given, if the infusion tube opposite-installation detection unit 92 detects that the infusion tube 72 is installed in the opposite direction, the warning message is also blinked on the display unit 80A and the speaker 603 also generates an alarm sound in order to notify surrounding personnel of the bubbles or the occlusion.

The detection of bubbles by the bubble detecting sensor 602 and the detection of occlusion by the occlusion sensor 91 are well-known and will thus not be described in detail. The structure and principle of the infusion tube opposite-installation detection unit 92 will be described below.

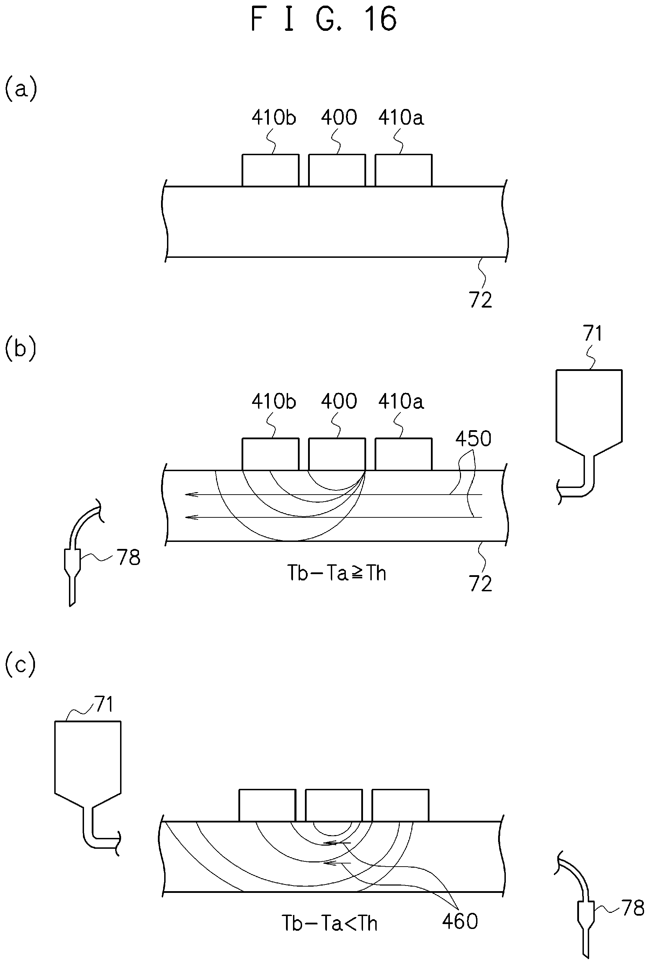

Fig. 16(a) shows the relation between the infusion tube opposite-installation detection unit 92 according to the second embodiment and the infusion tube. The infusion tube opposite-installation detection unit 92 according to the second embodiment comes into close contact with a heater 400 and a temperature sensors (thermistors) 410a and 410b when the infusion tube 72 is placed in the housing paths 84a, 84b, and 84c, as shown in Fig. 16(a). The second embodiment is designed such that a distance between the heater 400 and the temperature sensor 410a is the same as a distance between the heater 400 and the temperature sensor 410b. Furthermore, the heater 400 incorporates a thermostat, and once a preset target temperature is reached, maintains the temperature.

The infusion pump 70 according to the second embodiment feeds the fluid medicine in the tube 72 from right side to left side of Fig. 16(a). Thus, when it is assumed that the tube 72 from the infusion bag 71 is set in the infusion pump 70 in the correct direction as shown in Fig. 13 and the peristaltic fingers 83-1 to 83-5 are driven to start feeding the fluid medicine, the fluid medicine flows smoothly through the infusion tube 72 as illustrated by arrow 450 shown in Figure 16(b).

Thus, when the heater 400 is driven under heat, the heat propagates as shown by an illustrated circular arc, and the temperature sensor 410b positioned downstream of the heater 400 can detect an increase in temperature. However, the temperature sensor 410a positioned upstream of the heater 400 fails to detect the increase in temperature, or even if temperature sensor 410a can detect the rise, the degree of the increase is low. Hence, when temperatures detected by the temperature sensors 410a and 410b are denoted by Ta and Tb, respectively, Formula (1) holds true for a threshold Th.

$$Tb - Ta \geq Th \qquad (1)$$

On the other hand, it is assumed that feeding of a fluid medicine is started with the infusion tube 72 connected to the accommodation path 84 in the infusion pump 70 in the opposite direction in the lateral direction as shown in Figure 16(C) or that downstream occlusion has occurred. In this case, the infusion bag 71 is full of the fluid medicine and fixed to a position higher than the infusion pump 10. Thus, even when feeding of the fluid medicine by the infusion pump 10 is started, a flow velocity achieved by the infusion pump 70 is lower than a flow velocity in Fig. 16(b) or the flow may stop. Thus, in this state, when the heater 400 is driven to generate heat, the heat propagates as shown by a circular arc in Fig. 16(c). In this case, a difference in temperature between the temperature sensors 410a and 410b is smaller than a difference in Fig. 16(b). Thus, the following relation in Formula (2) is established.

$$Tb - Ta < Th \qquad (2)$$

Thus, it is understood that the opposite installation of the infusion tube or the downstream occlusion can be detected by driving the heater 400 to generate heat and detecting the temperature both on the upstream side and the downstream side of the heater.

The heater 400 and the temperature sensors 410a and 410b are fixed and arranged at equal intervals. Furthermore, when driven, the heater 400 generates a constant amount of heat per unit time. The volume of heated fluid medicine increases consistently with the flow rate. Thus, it should be noted that the threshold Th depends on a flow rate set by the operator (the amount of fluid medicine fed per unit time; also referred to as the flow velocity in the infusion tube). That is, the threshold Th may be arithmetically determined from the flow rate set by the operator or determined by referencing a pre-stored lookup table.

[Description of the process procedure]

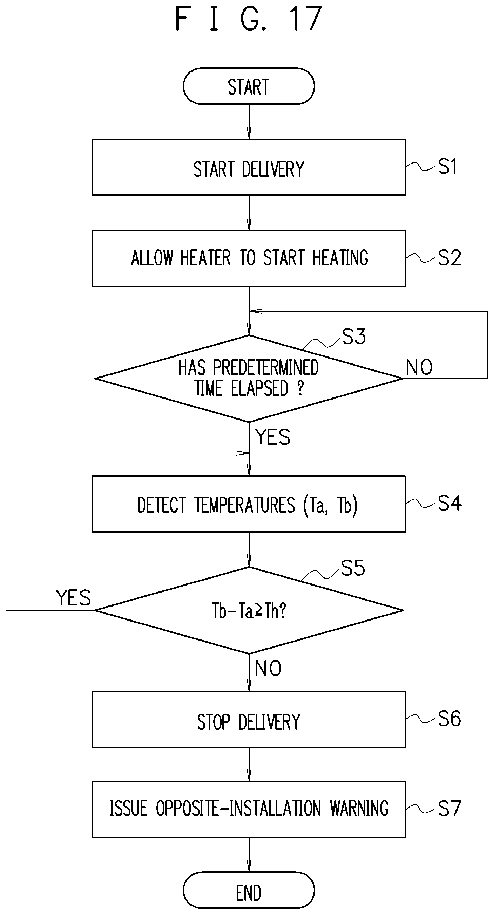

Now, a process procedure of the control unit 600 for the infusion pump 70 according to the second embodiment will be described with reference to a flowchart in Fig. 17. Fig. 17 shows processing carried out when a delivery start switch on the operation unit 80A is operated. Not only the infusion tube 71 has already been placed in the housing path 84 in the infusion pump 70 according to the second embodiment, but also a priming process and the input of the flow rate (the amount of fluid medicine fed per unit time) and the like have been finished, with the vein puncture needle 78 already introduced into the patient's vein. Furthermore, it is assumed in the description that the threshold Th has been determined in accordance with the set flow rate.

When the feeding start switch on the operation unit 80A is operated, the control unit 600 drives the driving unit 601 in accordance with the set flow rate to drive the peristaltic fingers 83-1 to 83-5, thus starting to feed the fluid medicine in the tube (step S1).

Subsequently, driving of the heater 400 in the infusion tube opposite-installation detection unit 92 is started to generate heat (step S2). As a result, temperature of the heater 400 increases, but a certain amount of time is needed for the temperature to reach a target temperature. Furthermore, a time T corresponding to the distance between the heater 400 and the temperature sensor 410b and the set flow rate needs to elapse before an amount of heat from the heater 400 reaches the temperature sensor 410b, located downstream, via the fluid medicine in the fluid medicine tube. Thus, to prevent the temperature sensors 410a and 410b from detecting the temperature until the above-described condition is sufficiently met, the control unit 600 waits until the time T elapses (step S3).

When the time T has elapsed since the start of heating by the heater 400, the process proceeds to step S4, where the temperature sensors 410a and 410b measure the temperatures Ta and Tb.

As a result of the measurement, the control unit determines whether a conditional expression "Tb - Ta ≥ Th" is met (step S5). Upon determining that the conditional expression is met, the control unit considers the infusion tube 77 to be set in the correct direction.

On the other hand, upon determining that the conditional expression "Tb - Ta ≥ Th" is not met, the control unit determines that the infusion tube 77 is housed in the opposite lateral direction in the housing unit 84 or that downstream occlusion is occurring, and thus stops driving of the peristaltic fingers 83-1 to 83-5 by the driving unit 601 to stop the delivery (step S6). Then, the control unit allows the display unit 80A to blink a warning message indicating that the infusion tube is installed in the opposite direction or that downstream occlusion is occurring, and allows the speaker 603 to generate an alarm sound, then the control unit thus ends the process (step S7). The warning notification in step S7 is provided both on the screen and in sound, but may be provided exclusively in one of these two ways. Moreover, if the infusion pump is connected to a network, the warning message may be communicated to a server.

As described above, according to the second embodiment, the infusion pump internally has the heat source that propagates heat through the infusion tube and the temperature sensors that detect the heat propagated to the fluid medicine in the infusion tube, and can thus detect the incorrect installation of the infusion tube in the infusion pump based on the degree of the propagation of the heat from the heat source during delivery. Therefore, the opposite-installation warning enables the operator to be urged to install the infusion tube in the correct direction.

According to the second embodiment, the warning is issued only when the opposite installation of the infusion tube is detected. However, until the opposite installation is detected or even though the infusion tube is determined to be correctly installed, a mark indicative of this condition may be displayed on the display unit 80B so as to notify the surrounding personnel of this condition. Furthermore, according to the second embodiment, the heat source is described as a heater. However, a heat source that reduces the temperature may be used. An example of the heat source that reduces the temperature is a Peltier element. The use of the heat source that reduces the temperature is different from the use of the above-described heat source in that, when the infusion tube is correctly housed in the housing path 84, the temperature detected by the downstream side temperature sensor is lower than the temperature detected by the upstream side temperature sensor (which detects a temperature close to room temperature).

[Third embodiment]

In the above description, the temperature sensors 410a and 410b located adjacent to and across the heater 400 measure the temperatures Ta and Tb, and the opposite installation of the infusion tube 72 in the infusion pump is detected based on the difference in temperature. In the second embodiment, a case will be described in which the opposite installation of the infusion tube 72 is detected by measuring a delay time in the propagation of the temperature.

The third embodiment is different from the first embodiment in the structure of an infusion tube opposite-installation detection unit 92 and a detection process carried out by the infusion tube opposite-installation detection unit 92. Thus, these differences will be described, and the remaining part of the configuration will not be described.

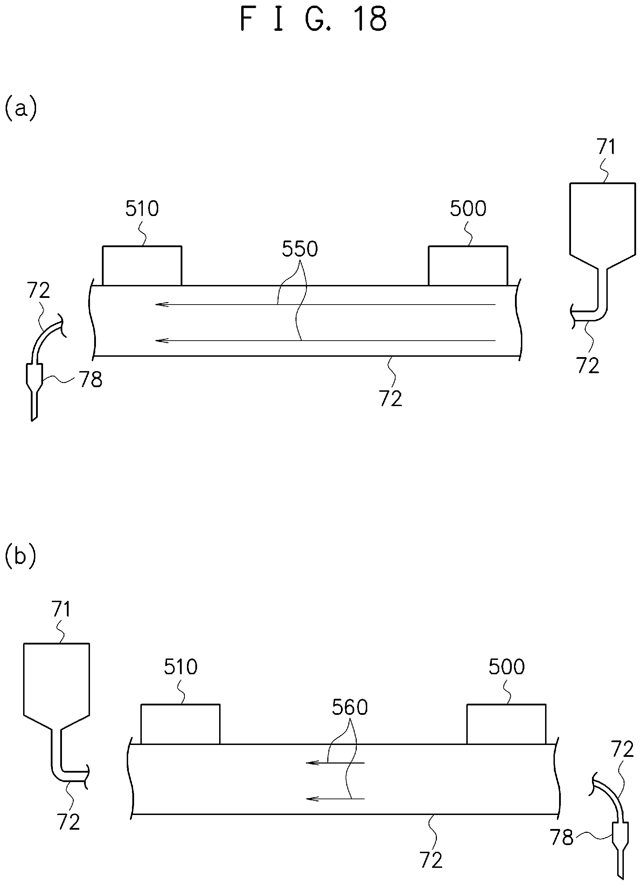

Fig. 18(a) shows a configuration of the infusion tube opposite-installation detection unit 92 according to the third embodiment. The infusion tube opposite-installation detection unit 92 includes a heater 500 and a temperature sensor 510 provided downstream of the heater 500 with respect to delivery carried out by an infusion pump. Fig. 18(a) is different from Fig. 16 in that only a single temperature sensor is provided and that the heater 500 and the temperature sensor 510 are located away from each other so as to accurately measure a delay time described below.

As shown in Fig. 16(a), when the infusion tube 72 is housed in a housing path 84 in the correct direction and a driving unit 601 is driven to carry out delivery, a fluid medicine flows smoothly as shown by an illustrated arrow 550. When the cross sectional area of a portion of the infusion tube 72 through which an infusion flows is denoted by S and a flow rate (the amount (volume) of fluid medicine fed per unit time) set for an infusion pump 70 is denoted by M, the flow velocity v of the fluid medicine is given by:

$$v = M/S.$$

When the distance between the heater 500 and the temperature sensor 510 is denoted by L, a time t0 needed for an amount of heat from the heater 500 to propagate to the temperature sensor 510 is given by:

$$t0 = L/V = L * S/M$$

However, even if the flow velocity is zero, the temperature propagates through the fluid medicine. The heat conductivity of the material of the infusion tube 72 needs to be taken into account. Thus, an adjustment value $\alpha$ involving these conditions is added to t0, and the result of this addition is defined as an allowable delay time T. The $\alpha$ may be derived based on sampling under various conditions and may be provided in a lookup table so as to be determined according to the flow velocity.

According to the third embodiment, the heater 500 is intermittently driven to generate heat. That is, a process of continuing heating for a predetermined time and then stopping the heating is repeated. Since delivery is being carried out, the temperature sensor 510 can detect the repetition of heating and non-heating as a variation in temperature. When one cycle of heating and the subsequent non-heating process is defined as a heating/non-heating process, the infusion tube 72 is considered to be housed in the housing path 84 in the correct direction when the temperature sensor 510, located downstream, can detect an increase in temperature greater than $\Delta C$ (preset threshold) with respect to the last measured temperature within the time T from completion of one heating/heating process. Subsequently, the heating/non-heating process is repeated.

On the other hand, when the infusion tube 72 is housed in the housing path 84 in the opposite direction as shown in Fig. 17(b), the flow velocity is lower than in the case of the correct installation as shown an illustrated arrow 560. However, an infusion bag 71 may fail to be installed above the infusion pump 70. Furthermore, immediately after the beginning of the delivery, the infusion bag 71 may have a certain housing space, and a possibility that the fluid medicine flows smoothly is undeniable. That is, even when the infusion tube is installed in the opposite installation, an increase in temperature may be detected within the time T in the state immediately after the beginning of the delivery. However, when a certain time elapses, the flow velocity gradually decreases, and the temperature sensor 510 fails to detect the increase in temperature even though the time T has elapsed. In this case, the infusion tube 72 may be determined to be installed in the opposite direction, and a warning process may be carried out.

[Description of the process procedure]

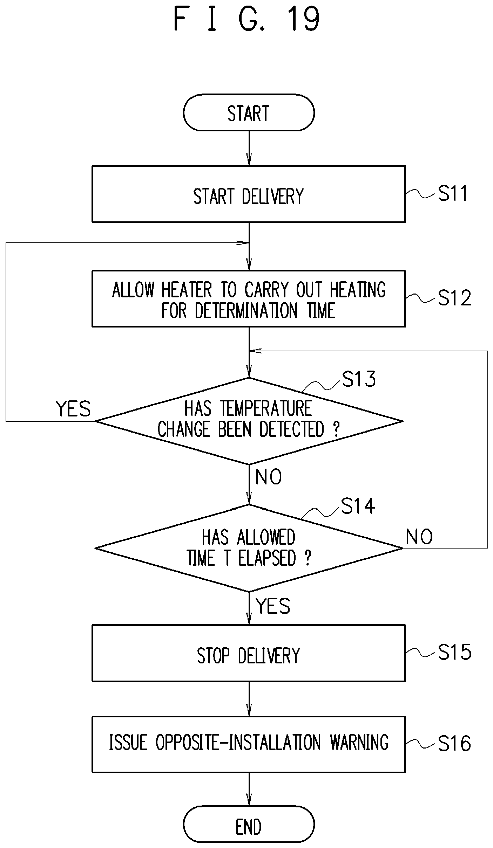

Now, a process procedure of a control unit 600 for the infusion pump 70 according to the second embodiment will be described with reference to a flowchart in Fig. 19. Fig. 19 shows processing carried out when a delivery start switch on an operation unit 80A is operated. Not only the infusion tube 71 has already been placed in the housing path 84 in the infusion pump 70 according to the third embodiment, but also a priming process and the input of the flow rate (the amount of fluid medicine fed per unit time) and the like have been finished, with a vein puncture needle 78 already introduced into the patient's vein. Furthermore, it is assumed in the description that the allowable delay time T has been determined in accordance with the set flow rate.

When a feeding start switch on the operation unit 80A is operated, the control unit 600 drives the driving unit 601 in accordance with the set flow rate to drive peristaltic fingers 83-1 to 83-5, thus starting to feed the fluid medicine in the tube (step S11).

Subsequently, a process is carried out which involves driving the heater 500 in the infusion tube opposite-installation detection unit 92 for a predetermined time so as to generate heat and stopping the heating when the predetermined time has elapsed (step S12). As a result, the fluid medicine is partly formed into a high-temperature portion, which flows downstream. The control unit 600 allows the temperature sensor 501 to measure the temperature and determines whether a difference from the last measured temperature is equal to or greater than $\Delta C$ (step S13). If the difference is not equal to or greater than $\Delta C$, the control unit 600 allows the heater to carry out a heating/non-heating process and then determines whether or not the allowable delay time T has elapsed (step S14). Upon determining that the allowable delay time T has not elapsed, the control unit 600 returns to step S13.

When, as a result of the above-described operation, a change in temperature (increase in temperature) can be detected within the allowable delay time T from completion of heating/non-heating by the heater 500, the result of the determination in step S13 is Yes, and the next heating/non-heating process is carried out.

On the other hand, if no change in temperature can be detected even after the allowable delay time T has elapsed, the delivery speed is not equal to the set flow rate and the control unit 600 determines that the infusion tube 72 is housed in the housing path 84 in the opposite lateral direction. The control unit 600 then stops driving of the peristaltic fingers 83-1 to 83-5 by the driving unit 601 in order to stop the delivery (step S15). Then, the control unit allows a display unit 80A to blink a warning message indicating that the infusion tube is installed in the opposite direction, and allows a speaker 603 to generate an alarm sound. The control unit thus ends the process (step S16). The warning notification in step S7 is provided both on the screen and in sound, but may be provided exclusively in one of these two ways. Moreover, if the infusion pump is connected to a network, the warning message may be communicated to a server.

As described above, the third embodiment allows the incorrect installation of the infusion tube in the infusion pump to be detected based on the degree of propagation of heat from the heat source to the fluid medicine during delivery.

The third embodiment has been described in conjunction with the detection of an increase in temperature. However, a decrease in temperature may be detected. Alternatively, both an increase and a decrease in temperature may be detected. In particular, when the temperature is actively reduced, the use of a Peltier element can make the temperature equal to or lower than room temperature, enabling improvement of the accuracy of detection of an increase and a decrease in temperature.

Reference Signs List

1, 70 Infusion pump
2 Main body cover
2A Upper portion of main body cover
2B Lower portion of main body cover
3 Display unit (an example of warning means)
4 Operation panel unit
5 Opening and closing cover
50 Tube installation unit (also referred to as a line installation unit)
51 Bubble detecting sensor
52 Upstream side occlusion sensor
53 Downstream side occlusion sensor
60 Delivery driving unit
80 User interface unit
80A Operation unit
80B Display unit
72 Infusion tube
71 Infusion bag
81 Lock/unlock lever

82 Tube clamp holding unit
83-1 to 83-5 peristaltic Fingers
84 Housing path
91 Occlusion sensor
92 Infusion tube opposite-installation detection unit
93 Pump mechanism
100 Control unit
131 Speaker (an example of warning means)
132 Buzzer (an example of warning means)
150 Infusion tube set direction display unit
151 Drug containing bag display unit
152 Patient side display unit
153 Delivery direction display unit
200 Infusion tube (also referred to as a infusion line)
200A Upstream side of infusion tube
200B Downstream side of infusion tube
T Correct direction
N Incorrect direction
X Horizontal direction (lateral direction)
400, 500 Heaters
410a, 410b, 510 Temperature sensors

### Claims

1. An infusion pump (1) comprising:

   a tube installation unit (50) in which an infusion tube (200) allowing a drug (171) in a drug containing bag (170) to be infused into a patient (P) is installed in use in a lateral horizontal direction; and
   a delivery driving unit (60) that, with the infusion tube installed in the lateral horizontal direction, applies peristaltic motion to the infusion tube to deliver the drug in a delivery direction (T) preset along the lateral horizontal direction,
   the tube installation unit comprising:

      a bubble detecting sensor (51) that detects a bubble generated in the infusion tube;
      **characterised in that** the tube installation unit further comprises:

         a downstream occlusion sensor (53) that detects a blocked state of the infusion tube; and
         a control unit (100) that determines that the infusion tube is set in a direction opposite (N) to the delivery direction based on a value of a downstream occlusion signal with a tendency toward an increase from the downstream occlusion sensor obtained when, with the infusion tube set in the direction opposite to the delivery direction, a clamp (179) for the infusion tube between the drug containing bag and the delivery driving unit is opened by the control unit to carry out priming, instead of driving the delivery driving unit (60), for filling the drug from the drug containing bag into the infusion tube to deliver the drug to the patient, the downstream occlusion signal indicating that an internal pressure of the infusion tube has increased.

2. The infusion pump according to claim 1, comprising warning means for issuing a warning in accordance with an instruction from the control unit when the control unit determines that the infusion tube is installed in the direction opposite to the delivery direction.

### Patentansprüche

1. Infusionspumpe (1), umfassend:

   eine Schlauchinstallationseinheit (50), in der ein Infusionsschlauch (200), welcher das Infundieren eines Arzneimittels in einem Arzneimittel-enthaltenden Beutel (170) in einen Patienten (P) ermöglicht, bei Verwendung in einer seitlichen horizontalen Richtung installiert ist; und
   eine Verabreichungsdruckeinheit (60), die, bei installiertem Infusionsschlauch in der seitlichen horizontalen Richtung auf den Infusionsschlauch eine peristaltische Bewegung ausübt, um das Arzneimittel in eine Verabreichungsrichtung (T) zu verabreichen, die entlang der seitlichen horizontalen Richtung vor-eingestellt ist, wobei die Schlauchinstallationseinheit Folgendes umfasst:

      einen Blasendetektierungssensor (51), der eine im Infusionsschlauch erzeugte Blase detektiert;
      **dadurch gekennzeichnet, dass** die Schlauchinstallationseinheit ferner Folgendes umfasst:

         einen Stromabwärts-Verschluss-Sensor (53), der einen blockierten Zustand des Infusionsschlauchs detektiert; und
         eine Steuerungseinheit (100), die bestimmt, dass der Infusionsschlauch in eine Richtung (N) eingestellt ist, die der Verabreichungsrichtung entgegengesetzt ist, und zwar auf Basis eines Werts eines Stromabwärts-Verschluss-Signals mit einer Tendenz zu einer Erhöhung von dem Stromabwärts-Verschluss-Sensor, der, wobei der Infusionsschlauch in die zur Verabreichungsrichtung entgegengesetzte Richtung eingestellt ist, dann erhalten wird, wenn eine Klemme (179) für den Infusionsschlauch zwischen dem Arzneimittel-enthaltenden Beutel und der Verabreichungsdruckeinheit durch die Steuerungseinheit geöffnet wird, um ein Ansaugen anstelle eines Druckausübens der Verabreichungsdruckeinheit (60) durchzuführen, um das Arzneimittel aus dem Arzneimittel-enthaltenden Beutel in den Infusionsschlauch zu füllen, um das Arzneimittel dem Patienten zu verabreichen, wobei das Stromabwärts-Verschluss-Signal anzeigt, dass ein Innendruck des Infusionsschlauchs angestiegen ist.

2. Infusionspumpe gemäß Anspruch 1, umfassend Warneinrichtungen zur Ausgabe einer Warnung gemäß einem Befehl von der Steuerungseinheit, wenn die Steuerungseinheit bestimmt, dass der Infusionsschlauch in die zur Verabreichungsrichtung entgegengesetzte Richtung installiert ist.

**Revendications**

1. Pompe de perfusion (1) comprenant :

une unité d'installation de tube (50) dans laquelle un tube de perfusion (200) permettant à un médicament (171) dans une poche de confinement de médicament (170) d'être perfusé dans un patient (P), est installé, à l'usage, dans une direction horizontale latérale ; et
une unité d'entraînement de distribution (60) qui, avec le tube de perfusion installé dans la direction horizontale latérale applique un mouvement péristaltique sur le tube de perfusion pour distribuer le médicament dans une direction de distribution (T) prédéterminée le long de la direction horizontale latérale,
l'unité d'installation de tube comprenant :

un capteur de détection de bulle (51) qui détecte une bulle générée dans le tube de perfusion ;
**caractérisée en ce que** l'unité d'installation de tube comprend en outre :

un capteur d'occlusion en aval (53) qui détecte un état bloqué du tube de perfusion ; et
une unité de commande (100) qui détermine que le tube de perfusion est placé dans une direction opposée (N) à la direction de distribution, en fonction d'une valeur du signal d'occlusion en aval avec une tendance à l'augmentation à partir du capteur d'occlusion en aval obtenue lorsque, avec le tube de perfusion placé dans la direction opposée à la direction de distribution, une pince (179) pour le tube de perfusion entre la poche de confinement de médicament et l'unité d'entraînement de distribution est ouverte par l'unité de commande pour réaliser l'amorçage, au lieu d'entraîner l'unité d'entraînement de distribution (60), pour amener le médicament de la poche de confinement de médicament dans le tube de perfusion afin de distribuer le médicament au patient, le signal d'occlusion en aval indiquant qu'une pression interne du tube de perfusion a augmenté.

2. Pompe de perfusion selon la revendication 1, comprenant un moyen d'avertissement pour émettre un avertissement selon une instruction provenant de l'unité de commande lorsque l'unité de commande détermine que le tube de perfusion est installé dans la direction opposée à la direction de distribution.

# F I G. 1

# F I G. 2

# F I G. 3

# F I G. 4

# F I G. 5

# FIG. 6

# F I G. 7

(A)

CORRECT

(B)

INCORRECT

# F I G. 8

## (A)

DELIVERY DIRECTION (T)

170
171
200A
179
N 200 53 171 60 50 52 51 199 171
200B

## (B)

DELIVERY DIRECTION (T)

170
171
200A
179
N 200 53 171 60 50 52 51
200B
BL G
P

# F I G. 9

SETTING ALONG CORRECT DIRECTION (T DIRECTION)

OCCLUSION DETECTING ~52  53

(A)

| OCCLUSION DETECTING SENSOR / OCCLUSION DETECTING STATUS | UPSTREAM OCCLUSION DETECTING SENSOR | DOWNSTREAM OCCLUSION DETECTING SENSOR |
|---|---|---|
| UPSTREAM OCCLUSION DETECTING (CASE 1) | TUBE INTERNAL PRESSURE HAS DECREASED | NO CHANGE (EXCEPT FOR VERY SMALL CHANGE) OR TUBE INTERNAL PRESSURE HAS SLIGHTLY DECREASED |
| DOWNSTREAM OCCLUSION DETECTING (CASE 2) | NO CHANGE (EXCEPT FOR VERY SMALL CHANGE) | TUBE INTERNAL PRESSURE HAS INCREASED |
| NORMAL CASE (CASE 3) | NO CHANGE (EXCEPT FOR VERY SMALL CHANGE) | NO CHANGE (EXCEPT FOR VERY SMALL CHANGE) |

179

SETTING ALONG INCORRECT DIRECTION (N DIRECTION)

~52  53

(B)

| OCCLUSION DETECTING SENSOR / OCCLUSION DETECTING STATUS | UPSTREAM OCCLUSION DETECTING SENSOR | DOWNSTREAM OCCLUSION DETECTING SENSOR |
|---|---|---|
| UPSTREAM OCCLUSION DETECTING (CASE 4) | NO CHANGE (EXCEPT FOR VERY SMALL CHANGE) | TUBE INTERNAL PRESSURE HAS INCREASED |
| DOWNSTREAM OCCLUSION DETECTING (CASE 5) | TUBE INTERNAL PRESSURE HAS DECREASED | TUBE INTERNAL PRESSURE HAS SLIGHTLY INCREASED |
| NORMAL CASE (CASE 6) | NO CHANGE (EXCEPT FOR VERY SMALL CHANGE) | TUBE INTERNAL PRESSURE HAS INCREASED |

189

# F I G. 10

EP 2 700 424 B1

# F I G. 11

IN PRIMING MODE, BUBBLE DETECTING SENSOR
HAS CONTINUOUSLY REACTED

(A)

300

PRIMING ERROR : ⚠

CHECK WHETHER
INFUSION LINE IS SET
IN OPPOSITE DIRECTION

~3

IN PRIMING MODE, DOWNSTREAM OCCLUSION DETECTING SENSOR
HAS CONTINUOUSLY REACTED

(B)

301

PRIMING ERROR : ⚠

CHECK WHETHER
INFUSION LINE IS SET IN OPPOSITE
DIRECTION OR WHETHER DOWNSTREAM
OCCLUSION DETECTING IS OCCURRING

~3

DURING NORMAL DELIVERY, DOWNSTREAM OCCLUSION DETECTING SENSOR
HAS CONTINUOUSLY REACTED

(C)

302

DELIVERY ERROR : ⚠

CHECK WHETHER
INFUSION LINE IS SET IN OPPOSITE
DIRECTION OR WHETHER DOWNSTREAM
OCCLUSION DETECTING IS OCCURRING

~3

# F I G. 12

# F I G. 13

# F I G. 14

# F I G. 15

OCCLUSION DETECTING SENSOR — 85

BUBBLE DETECTING SENSOR — 602

INFUSION TUBE OPPOSITE-INSTALLATION DETECTION UNIT — 86
HEATER
TRMPERATURE SENSOR

SPEAKER — 603

CONTROL UNIT — 600

USER INTERFACE UNIT — 80
OPERATION UNIT — 80A
DISPLAY UNIT — 80B

DRIVING UNIT — 601

PERISTALTIC FINGER
83-1  83-2  83-3  83-4  83-5

EP 2 700 424 B1

# F I G. 16

(a)

410b   400   410a

72

(b)

410b   400   410a   450

71

78

$Tb - Ta \geqq Th$

72

(c)

71

78

$Tb - Ta < Th$   460

# F I G. 17

START

START DELIVERY ~S1

ALLOW HEATER TO START HEATING ~S2

HAS PREDETERMINED TIME ELAPSED ? —— S3
NO

YES

DETECT TEMPERATURES (Ta, Tb) ~S4

Tb−Ta≧Th? —— S5
YES
NO

STOP DELIVERY ~S6

ISSUE OPPOSITE−INSTALLATION WARNING ~S7

END

# F I G. 18

(a)

(b)

# F I G. 19

```
                    ┌─────────────┐
                    │    START    │
                    └─────────────┘
                           │
                           ▼
              ┌────────────────────────┐
              │     START DELIVERY     │──S11
              └────────────────────────┘
                           │
        ┌──────────────────┤
        │                  ▼
        │     ┌────────────────────────────┐
        │     │ ALLOW HEATER TO CARRY OUT   │──S12
        │     │ HEATING FOR DETERMINATION   │
        │     │ TIME                        │
        │     └────────────────────────────┘
        │                  │
        │                  ▼
        │            ╱──────────╲        S13
      YES          ╱ HAS TEMPERATURE ╲
        └────────╱ CHANGE BEEN DETECTED ?╲
                  ╲                ╱
                   ╲──────────────╱
                         │ NO
                         ▼
                   ╱──────────╲         S14
                  ╱ HAS ALLOWED ╲      NO
                 ╱ TIME T ELAPSED ?╲────┐
                  ╲               ╱     │
                   ╲─────────────╱      │
                         │ YES
                         ▼
              ┌────────────────────────┐
              │     STOP DELIVERY      │──S15
              └────────────────────────┘
                         │
                         ▼
        ┌──────────────────────────────────────┐
        │ ISSUE OPPOSITE-INSTALLATION WARNING   │──S16
        └──────────────────────────────────────┘
                         │
                         ▼
                    ┌─────────┐
                    │   END   │
                    └─────────┘
```

**EP 2 700 424 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010200775 A

- GB 2312046 A